# EUROPEAN PATENT APPLICATION

(11) **EP 2 808 039 A1**
(43) Date of publication of application: **03.12.2014**
(21) Application number: 13169393.9
(22) Date of filing: 27.05.2013
(51) Int. Cl.: A61L 27/04, A61L 27/06, A61L 27/30, A61L 27/54

(54) **Dental implants for high risk patients**

(71) Applicant: Straumann Holding AG, 4002 Basel (CH)
(72) Inventor: Walter, Martin Sebastian, 86424 Dinkelscherben (DE); Frank, Matthias Johannes, 83666 Waakirchen (DE); Haugen, Håvard Jostein, 0376 Oslo (NO)
(74) Representative: Valea AB

(57) **Abstract**

The invention relates to medical and dental prosthetic devices and implants having improved properties for promoting soft tissue healing in high-risk patients. The present invention concerns dental prosthetic devices and implants partially and/or fully coated with covalently bound doxycycline. The invention relates to medical and dental prosthetic devices and implants having improved properties for promoting soft tissue healing. The invention further relates to the use of Doxycycline for promoting soft tissue healing and/or preventing inflammation and/or infection in soft tissue in proximity to a medical prosthetic device or implant deployed in the body of a patient belonging to a group high-risk patient group.

## Description

### FIELD OF THE INVENTION

The invention relates to medical and dental prosthetic devices and implants having improved properties for promoting soft tissue healing. The present invention concerns dental prosthetic devices and implants partially and/or fully coated with covalently bound doxycycline. The invention further relates to the use of Doxycycline for promoting soft tissue healing and/or preventing inflammation and/or infection in soft tissue in proximity to a medical prosthetic device or implant deployed in the body of a patient belonging to a group high-risk patient group.

### BACKGROUND OF THE INVENTION

### Dental implants

Improving techniques of implant production and broadening knowledge about the important factors for implant success, have led to success rates of dental implants well above 90 %. In that respect, diabetic patients, for example, are not seen as contraindicated for dental implants anymore, but rather that a controlled state of the disease is required for successful implantation. Despite these improvements, there are still patient groups that have poor success rates with commercially available implants due to smoking habits, radiation therapy or poor oral hygiene. Furthermore, an increasing number of elderly patients prefer dental implants and implant-retained partial dentures over conventional dentures, creating larger and more complex patient groups. The need for improved dental implants in these patient groups is caused by manifold factors, including general poor bone density, wound healing disruptions or high risk for infections.

For this reason a large proportion of scientific effort has been directed towards treatments that can increase the chances of implant success in poor quality bone.

In the case of diabetes, but also tobacco use, studies suggest, that systemic application of antibiotics could improve the success rates of dental implants for these patients. The factors that have to be controlled in this context are the stimulation of bone growth around the dental implant for improved osseointegration and the risk of infection at the implant site.

### Success of dental implants

The basic criteria for implant success are generally held to depend on the biocompatibility of the implant material, the macroscopic and microscopic nature of the implant surface, the status of the implant bed in both a health (non-infected) and a morphological (bone quality) context, the surgical technique used, the undisturbed healing phase and the subsequent prosthetic design and long-term loading phase.

Nearly all developments that lead to improved implant success rates up to the present were focused on at least one of these factors. The requirements can be further divided in factors that can be controlled by technology, covering treatment method and material used and factors that are given by the patients' condition before, during and after implantation. By far the most effort has been taken to optimize the factors that can be controlled by technology, *such* as the biocompatibility of the implant material, the macroscopic and microscopic nature of the implant surface.

### Titanium and its alloys as implant material

Titanium is the material most commonly used today to achieve good biocompatibility and sustainable implant success. The biocompatibility of titanium and its superior properties as an implant material was held to be connected to the 2 - 10 nm thick oxide layer, instantly formed on titanium in the presence of oxygen. Due to this oxide layer, titanium features high polarization resistance, protecting the metal against corrosion and therefore hinders the release of metallic ions into the human body. As a result of titanium oxide's high dielectric constant, the surface oxide film was found to be an attractive site for the establishment of chemical bindings and the attachment of a large spectrum of doxycyclines. This interaction is also the reason why the placement of titanium in bone results in the establishment of a strong bone-implant interface. The establishment of this strong connection between the living bone and tissue and the histological proof thereof is called osseointegration.

Despite the successful application of titanium implants, research constantly aimed on the development of advanced alloying techniques for titanium to optimize biocompatibility and mechanical properties. One of the most recent innovations, the alloying of titanium with zirconium, showed broad success and acceptance. As titanium and zirconium are the only metals that do not show osteoblast growth inhibition, a combination of both is well suited for implants. A significantly higher removal torque and bone area could *in vivo* be shown for a titanium zirconium alloy compared to commercial pure (cp) titanium. Furthermore, it was observed that the oxides on titanium zirconium alloy surfaces are more stable and therefore favorable in terms of corrosion resistance. Also the alloying of titanium with zirconium improves the mechanical strength especially for applications in small diameter implants. While the mechanical strength is high for titanium zirconium alloys, they are well suited for implantation in cortical bone due to a low young's modulus, which prevents stress shielding. In addition, the mechanical properties of titanium zirconium alloys allow the placement of small-diameter implants in critical implantation sites, such as the front of the lower jaw where bone is scarce and the crestal bone is thick.

### Surface modification

There are a variety of surface treatments, which are commonly applied for improvement of surface properties of dental implants. Applied techniques can be divided in physiochemical modifications that change the surface topography, morphology and chemistry. Furthermore, additive processes are known to have potential to improve the physiological reaction of implants.

Morphology and topographical surface modifications have the potential to improve the interaction between implant and tissue. In this context, rough implant surfaces were found to promote osseointegration more than smooth surfaces. Mechanical and chemical methods or a combination of both can be used to optimize the surface morphology and topography. Especially blasting with titanium dioxide (TiO2) particles, etching and multistep etching are frequently used techniques.

Yet, hardly any modification of the surface morphology can be done without inducing changes of the chemical surface composition and vice versa. The etching processes used on titanium for surface modifications increases the amount of hydrogen on the Ti surfaces, as liberated hydrogen ions are attached to titanium's outer surface layer in the form of titanium hydride. This process can be influenced by molar strength of the acid and the etching time. Several studies suggest that the hydrogen content induces faster healing and furthermore better osseointegration. Therefore cathodic polarization (hydridation) has recently been applied to increase layer thickness and concentration of titanium hydride]. In addition the hydridation process can be used for attachment of ampholytic biological molecules that bind on the surface during hydridation together with hydrogen.

As the oxide layer on titanium is the most prominent feature of the material, modifications of this layer were tested. Yet, the approaches to increase titanium's biocompatibility by sheer increase of oxide layer thickness by anodic oxidation (hydroxylation) in acidic solutions did not show increased biocompatibility. Nevertheless, if hydroxylation is used with alkaline solutions, an increase of hydroxide (OH) groups on the surface can be achieved.

### Biomolecules

A variety of substances would qualify for the creation of bioactive surfaces. The underlying principle of their functionality is always a certain, beneficial stimulation of the host response by the applied substance. Therein the goal is the attachment of bioactive substances like proteins, enzymes or peptides on the surfaces. Cell attachment factors like fibronectin could improve attachment and spreading of cells on the implant surfaces, while the application of growth factors like bone morphogenetic proteins could directly influence the development of osteoblasts on the surfaces. The theoretical possibilities of suitable molecules are extensive, yet limited by the conditions that molecules are exposed during, the coating, solubility and degradation problems or simply destruction due to the attachment process. Furthermore a polarization of the molecule (ampholyte) is required for attachment in an electrochemical process.

As the basic criteria for implant success are generally held to depend on the biocompatibility of the implant material, measured on its effectiveness in promoting and/or stimulating osseointegration, most biomolecules tested today are selected for and evaluated on their effect on hard tissues.

Nearly all developments that lead to improved implant success rates up to the present have thus been focused on employing biomolecules stimulating hard tissue formation.

### Doxycycline

The semi-synthetic broad spectrum antibiotic Doxycycline belongs to the group of tetracycline antibiotics and is used for treatment of various infections and works by the inhibition of bacterial protein biosynthesis. In vitro studies suggest the efficiency of systemic Doxycycline application for bone growth promotion and treatment of periodontal disease as well as peri-implantitis. Furthermore, the applicability of Doxycycline for the control of osteogenic differentiation in genetically engineered mesenchymal stem cells has been proven. The administration of Doxycycline for periodontal disease and peri-implantitis treatment has so far been tried using topical drug delivery systems, such as gels.

Both systemic and topical administration of doxycycline has drawbacks, though; systemic administration of an antibiotic inadvertently leads to systemic interference with the patient's body and necessitates a higher overall dosage regime than topical application. Topical administration to a dental implant in particular, can only be performed during the placing of the implant or with post implant surgery, as the implant is more or less fully incorporated into the patient's body. What is more, topical administration of doxycycline with a carrier, such as a gel or a matrix, generates a fast release of the active substance, and it also necessitates that said carrier is biodegradable and thus the timespan at which doxycycline will be available to the surrounding tissue of the implant is limited.

In consequence, it is sought for an administration which will lead to a dosage optimization of the area of drug administration to the tissue directly surrounding the implant. For a combined use of Doxycycline with dental implants, a direct and permanent incorporation of this drug with the implant system would be favorable. Despite the potential benefits, though, successful binding of Doxycycline directly to an implant has not yet been reported in the literature.

The present inventors for the first time demonstrate covalent binding of Doxycycline hyclate onto a titanium zirconium (TiZr) alloy surface. It was surprisingly found that a hydrogen rich surface, as produced under cathodic reduction in acidic solutions, is suitable and can be more efficiently used for the attachment of doxycycline.

Further, the bioactivity of the biomolecule on the TiZr surface was assessed under in vitro and in vivo conditions and it was demonstrated that doxycycline coated dental implants surprisingly improved not only the biocompatibility of the implant measured on improved osseointegration, but clearly were able to promote healing of the soft tissue in proximity to the placed implant.

### DISCLOSURE OF THE INVENTION

Even though modern implants show high success rates, it is still challenging to successfully treat certain groups of patients with the implant systems available. Larger and more heterogeneous patient groups add to the complexity that implantology faces nowadays.

The present invention for the first time shows an improved craniofacial and/or dental prosthetic device or implant, containing a metal material wherein surface parts of the metal material (A) are coated one or more doxycycline(s) (C) associated therewith.

In one embodiment, the present invention for the first time shows the efficiency of a dental metal implant surface, such as a titanium zirconium surface, coated with doxycycline (Doxy), wherein the implant is coated using an electrochemical method in a cathodic reduction setup under acidic conditions.

Doxycycline coated implants were shown to surprisingly improve not only the biocompatibility of the implant measured on improved osseointegration, but especially were able to promote healing of the soft tissue in proximity to the placed implant. It is thus concluded that doxycycline has the potential to trigger bone growth around the implantation site, and at the same time reduce the risks of poor healing, infection and/or inflammation of the soft tissue in close proximity to the placed implant, such as in the gum tissue. Thus, for the first time, an improved craniofacial and/or dental prosthetic device or implant is disclosed that can be implanted even into high-risk patient groups, such as patients with diabetes, smoking habits, poor bone density, wound healing disruptions and/or high risk for infections.

Doxycycline is a semi-synthetic broad spectrum antibiotic, from the group of tetracycline antibiotics, which is used for treatment of various infections and works by inhibition of bacterial protein biosynthesis. Tetracycline is considered to enhance bone formation mostly on the basis of general knowledge about interaction with collagen formation and calcium incorporation. It has been proven to be efficient *in vitro* in application for bone growth promotion and treatment of periodontal diseases. Other studies could furthermore verify the applicability of doxycycline for the control of osteogenic differentiation in genetically engineered mesenchymal stem cells.

Administration of doxycycline for periodontal disease and peri-implantitis treatment is currently mainly done by application of drug delivery systems. Yet, a combined use of doxycycline with dental implants and its direct incorporation in the implant system has herein for the first time been shown to be favorable, as local administration reduces interference with the patient's body and optimizes the area of drug administration to the bone as well as the soft tissue directly surrounding the implant. The present inventors for the first time demonstrate successful binding of doxycycline directly to an implant.

Implant surfaces exposed to biological environments trigger a reaction based on the adsorption of water, ions, proteins and biomolecules. The adsorption of these molecules has an effect on cell attachment to the surface and therefore affects the tissue response. As the chemical properties of the surface directly influence the amount and composition of proteins adsorbed to the surface, coating with antibiotic agents can be used as one form of chemical surface modification. However, binding of biomolecules to titanium's native TiO₂ layer is difficult due to the low reactivity of this oxide layer.

The dental implant surfaces coated in the present invention are based on a titanium zirconium alloy with grit blasted and acid etched surface, which also served as control. The validation of the coating was done with FTIR, XPS, SIMS and Field emission SEM and its effect in vitro was tested on a MC3T3-E1 murine osteoblastic cell line. A rabbit in vivo study was executed to analyze bone marker gene expression and bone growth in critical size defect.

The doxycycline molecule was found intact on the surface and quantified using UV-VIS and FTIR, showing a mean concentration of 141 µg/cm² of Doxycycline on the samples. Inclusion of Doxycycline was documented up to a depth of approximately 0.44 µm by tracing the ¹²C signal in a SIMS analysis. The in vitro study with murine osteoblasts showed significantly increased alkaline phosphatase and osteocalcin gene expression levels after 14 days along with low cytotoxicity. Furthermore the in vivo study showed increased RNA, Coll-I and BMP2 expression at 8 weeks of healing.

The present inventors for the first time were able to apply a cathodic reduction process for the binding of Doxycycline to a TiZr surface. As the sample surface was negatively charged under cathodic reduction, the broad spectrum antibiotic Doxycycline was suitable for attachment due to its positive polarization. Doxycycline has several protons that are suitable to bind to the surface like the O-H proton and the A-ring amide. Therefore, it was assumed that Doxycycline would bind instead of the hydrogen formed under cathodic reduction as documented in the literature. Doxycycline was furthermore chosen with the prospect of creating a novel dental implant surface for treatment of critical implantation sites and contraindicated patients with low bone density.

Even though color change was detected on the coins in the experiments performed, the process did not alter Doxy, as there were no elevated temperatures employed in the process that could trigger degradation. As tetracyclines generally have many potential metal binding sides, a color change due to the binding of liberated metal ions was another possible explanation for the color change. The activity of the molecule was examined by an in vitro study and the integrity was investigated in FTIR.

The FTIR analysis showed that the Doxycycline molecule was present and intact on the surface. The concentration of the coating was analyzed with respect to the peak at 1454 cm⁻¹ and 1613 cm⁻¹ as those were described in the literature and were most prominent in our observations.

A strong peak at 1459 cm⁻¹ was from a CH₂ bending vibration of the aromatic amine compound N(CH₃)₂ in Doxy, which was also confirmed in the literature. Furthermore, a secondary amide N-H bending vibration of the amide II band was detected at 1555 cm⁻¹. This structure was rather surprising, as pristine Doxycycline should not contain that compound. The peak at 1575 cm⁻¹ was assigned to N-H bending of the aromatic ring A of Doxycycline. Another peak which was visible in both spectra positioned at 1611 cm⁻¹ was derived from C=O stretching on ring A. Considering that the peak at 1651 cm⁻¹ only appeared in the coated and spiked coated samples, it was likely that this peak was shifted from the peak at around 1660 cm⁻¹ of the pure Doxy. This peak was related to the amide I band of the primary amide with its C=O stretching vibration and was observed on the samples in agreement with the literature. Due to the peak shift, the amide was considered a possible binding site as the shifting of carboxyl peaks can occur due to changed hydrogen bonding state.

To analyze the exact amount of Doxycycline on the surfaces, a release study was executed. The total amount of 140.97 µg/cm² released over the seven days period was approximately three times higher than the amount calculated with the FTIR. This was attributed to general inaccuracies of the quantification of compounds in FTIR, as well as the influence of the rough surface and limited penetration depth. Even though no further measurements were conducted, it was assumed that after seven days all Doxycycline was released from the samples.

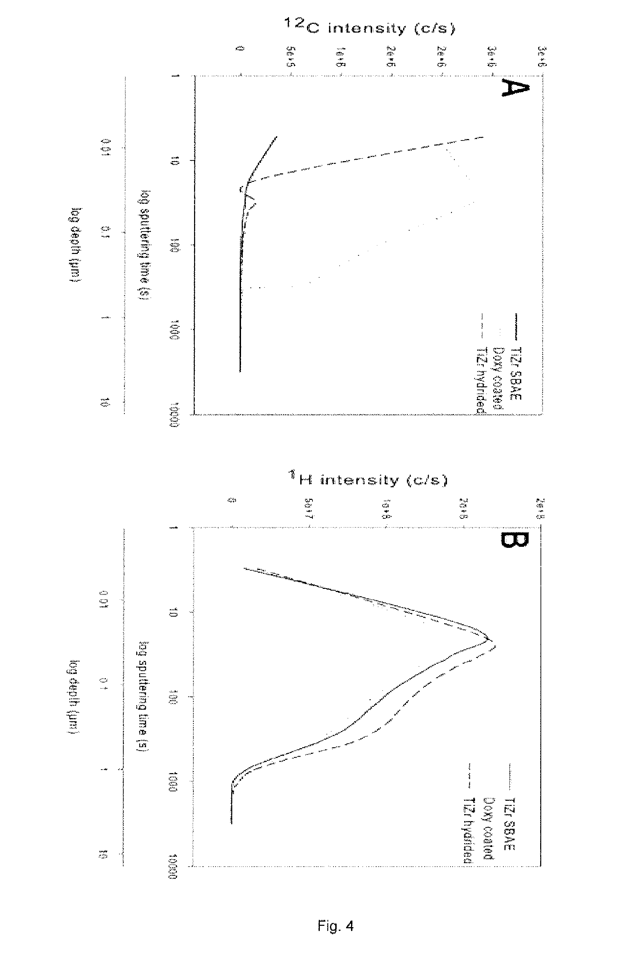

Beyond the detection of Doxycycline on the surface, an analysis of the molecules depth integration in the material was performed by SIMS (Figure 4). As Doxycycline contains a significant amount of carbon, tracking of the carbon ¹²C isotopes depth profile was used to track Doxycycline depth integration in the sample. This method was validated by analysis of a hydrided TiZr sample, to account for the possible carbon contamination, from the buffer solution during the process. As this contamination was negligible in terms of depth integration, ¹²C must have been derived from Doxycycline (Figure 4 A). Furthermore samples were protected under cover gas to exclude contamination with carbon from other sources and a TiZr SBAE reference sample for assessment of carbon in the source material was used. Based on the experience of previous work, it was believed that the binding of Doxycycline was linked to the buildup and remodeling of the hydrogen layer on the surface (Figure 4 B). Nevertheless, no elevated hydrogen levels were found for the Doxycycline coated samples which suggested that the hydrogen layer was remodeled during the process.

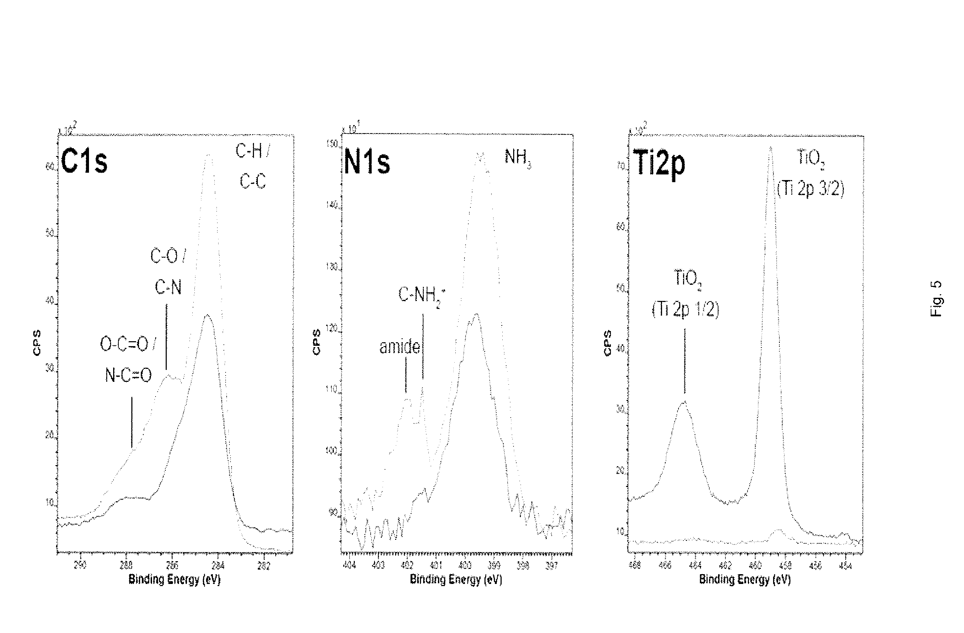

The XPS carbon peaks at 286 eV and at 287.5 eV were found to be corresponding to C-O / C-N and O-C=O / N-C=O respectively. As observed in figure 5, Ti2p intensity decreased strongly over the coating process, which was a clear signal for the coverage of the original surface. The titanium signal in both samples related to TiO₂. The largest peak of nitrogen at 399.5 eV, was attributed to NH₃ according to Moulder et al. Another peak of nitrogen, which was present on both samples at 401.6 eV was probably a C-NH₂⁺ compound. Furthermore the Doxycycline coated sample had a nitrogen peak at 402 eV, which was not observed on TiZr SBAE. This peak corresponded to amide and amino groups. Furthermore the literature assigned that region of the N1s peak to NH₄⁺. The fact that nitrogen was found on the control samples as well could have been due to the nitrogen cover gas packaging or relics from the processing history of the sample. Nevertheless, the appearance of the peak at 402 eV and the clear increase of nitrogen in general ascertained the presence of Doxycycline on the samples surface.

The XPS and FTIR analysis confirmed the presence of Doxycycline on the sample surface. The clear signal of the amide bands in FTIR and XPS and the appearance of surface masking, as indicated by the decrease of the titanium signal from the XPS, showed the presence of the molecule on the surface. Furthermore the FTIR measurements demonstrated that the molecule was intact.

The mechanism of Doxycycline attachment to the surface was most likely related to hydrogen bonding in a similar manner as described by Millenbach et al. and Wilhelmsen et al. (Millenbach P, Givon M. The electrochemical formation of titanium hydride. J Alloys Compd. 1982;87(2):179-84. Wilhelmsen W, Grande AP. Electrochemical and SIMS studies of cathodically formed hydride layers on titanium. Electrochim Acta.35(11-12):1913-7.). Potential binding sides could have been the amide group in ring A of Doxycycline. As protonation of Doxycycline occurs in the pH 1 - 6 region, it was likely, that a binding site became available on the amide group of Doxycycline in the form of a zwitterion on the primary amide complex. This could have triggered the reaction of Doxycycline molecules with each other in the powder form, but also the attachment of the biomolecule to the implant surface in the cathodic reduction. Therefore it is possible that Doxycycline was attached to a complex layer of hydrogen and oxygen and the molecule was actively bound to it at one or more binding sites. This mechanism would be similar to the one described in a paper on proline adsorption on titanium due to a zwitterion binding. The theory would sufficiently describe the binding mechanism and explain the appearance of the FTIR peak at 1555 cm⁻¹ which is related to a secondary amine. Furthermore it would explain the peak shift observed on Doxycycline powder from 1662 cm⁻¹ to 1651 cm¹ on Doxycycline coated samples.

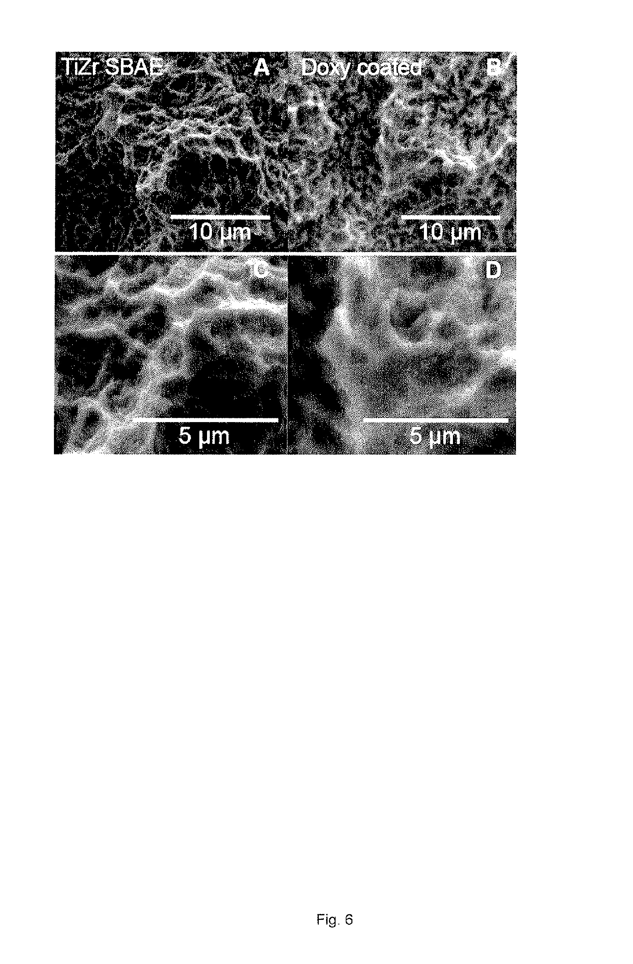

As seen in figure 6, the micro topography of both surfaces was similar, yet, the Doxycycline coated sample seemed to be smoothened in comparison to TiZr SBAE. Both surfaces of the present study were well suited for mechanical bone anchoring. Yet, this parameter is of greater importance for bone interlocking than for cell attachment. The smooth edges on the sub-micron scale and the white spherical structures observed on the Doxycycline coated sample were important in terms of their effect for cell attachment. It was assumed that the rough surface on the TiZr SBAE base material was covered with a film containing Doxy, gradually built during the coating. This coating smoothed the surface in the Doxycycline coated group and concealed the rough structures created by the etching process. Nevertheless, the optimal surface nano topography for cell attachment is unknown and always depends on both, surface topography and chemistry.

As the two analyzed surfaces exhibited lower cytotoxicity than the reference tissue cultured plastic and differences between the groups were marginal, a cytotoxic effect of Doxycycline or any side products could be excluded. After 14 days of cell culture ALP and OC were found significantly increased on MC3T3-E1 cells seeded on Doxycycline coated samples, while Coll-I was lower at the same time. The high expression of ALP and OC on the Doxycycline coated samples was an indication that the osteoblastic cells on these surfaces were in the mineralization phase, which points to fast differentiation. These results were in accordance with the literature and represented a typical effect of Doxycycline increasing osteoblast differentiation (Park JB. Low dose of doxycyline promotes early differentiation of preosteoblasts by partially regulating the expression of estrogen receptors. J Surg Res. 2012;178(2):737-42.; Park JB. Effects of doxycycline, minocycline, and tetracycline on cell proliferation, differentiation, and protein expression in osteoprecursor cells. J Craniofac Surg. 2011;22(5): 1839-42.; Piattelli A, Scarano A, Corigliano M, Piattelli M. Effects of alkaline phosphatase on bone healing around plasma-sprayed titanium implants: a pilot study in rabbits. Biomaterials. 1996;17(14):1443-9.; Sunaric S, Mitic S, Miletic G, Pavlovic A, Naskovic-Djokic D. Determination of doxycycline in pharmaceuticals based on its degradation by Cu(II)/H2O2 reagent in aqueous solution. J Anal Chem. 2009;64(3):231-7.)

The in vivo model used in this study was chosen due to its capacity to project the response in humans.

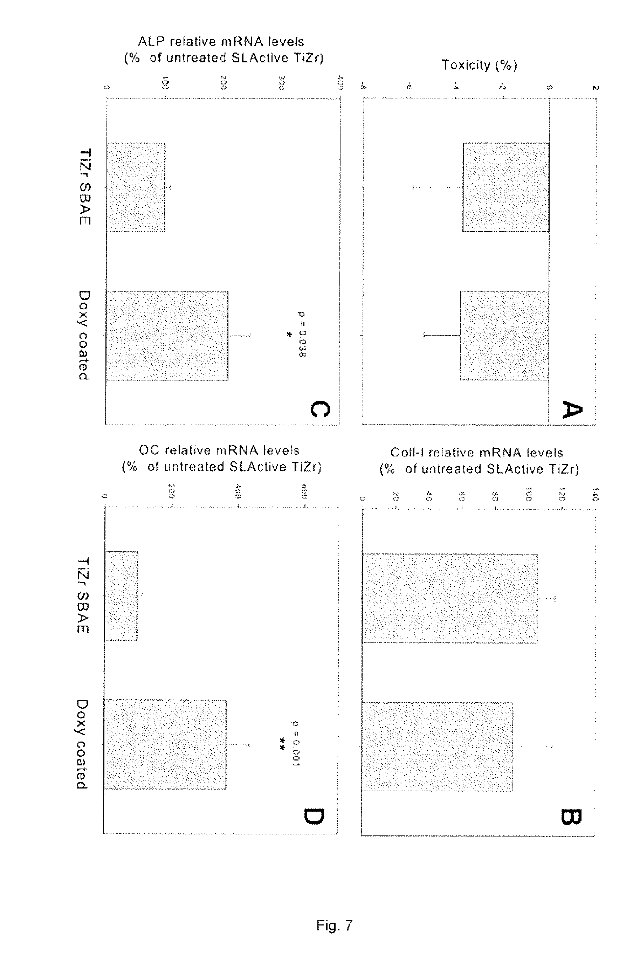

Inflamed bone tissue is known to release lactate dehydrogenase after cell death. The increase in cytotoxicity by assessment of LDH activity in the Doxycycline coated group after 8 weeks was interpreted as a late tissue response of bone remodeling. This assumption was furthermore supported by the low LDH activity observed for the Doxycycline coated group after 14 days in vitro (Figure 7).

Alkaline phosphatase is an early marker of osteoblast growth and its expression declines before the mineralization phase. As the relative ALP activity was significantly decreased after 8 weeks for both, hydrided and Doxycycline coated groups, the wound fluid analysis showed a late differentiation stage. A significant increase of total RNA content after 8 weeks, as well as the significantly higher Coll-I mRNA levels for the Doxycycline coated group showed the bioactivity of the surface coating with Doxycycline and indicate a proliferative stage. Coll-I is the main organic contributor to the mechanical strength of bone and induces high density bone formation. This was in accordance with the high BMP-2 gene expression levels in the Doxycycline coated group after 8 weeks, which is relevant for ossification and directly influences bone formation. The increased activity of osteocalcin in the Doxycycline coated group furthermore indicated the maturation of bone tissue organization after 8 weeks.

Bone resorption due to remodeling of bone by osteoclasts is connected to the mRNA levels from H⁺-ATPase and TRAP. While H⁺-ATPase had highest expression after 8 weeks in the Doxycycline coated group, TRAP was lowered in the Doxycycline coated group compared to TiZr SBAE at 8 weeks. This behavior could indicate the presence of a resorption / deposition state due to bone remodeling. The significant increase of pro-inflammatory IL-6 was countered in the present study by the presence of increased, anti-inflammatory IL-10 mRNA levels in the Doxycycline coated group. A conclusive remark on the inflammation status of the peri-tissue is therefore difficult.

The devolution of the 2 dimensional BMD showed the most significant results for the Doxycycline coated group. Its high depth integration suggested the release of the molecule from the implant and its transport through the defect. The Doxycycline coated group was besides the high BMD, promising due to the high BMD at the end of the volume of interest.

After 8 weeks of healing time increased healing of the Doxycycline coated group compared to all other reference groups was observed. The Doxycycline coated group showed higher values of bone density, bone volume was furthermore increased. The high bone surface / tissue ratio furthermore suggested the presence of a highly porous bone structure with large quantities low diameter trabecular bone. This theoretical analysis was also confirmed by the 3D images.

### DETAILED DISCLOSURE

The present invention relates a craniofacial and/or dental prosthetic device or implant, containing a metal material (A) selected from the group consisting of titanium or an alloy thereof, zirconium or an alloy thereof, tantalum or an alloy thereof, hafnium or an alloy thereof, niobium or an alloy thereof and a chromium-vanadium alloy, wherein surface parts of the metal material (A) are coated one or more doxycycline(s) (C) associated therewith, for use in high-risk patient groups.

In one embodiment, the present invention relates to a hydrided metal dental implant which is partially and/or fully coated with covalently bound Doxycycline(s) and which displays an improved in-healing in soft tissue. It was surprisingly found that a hydrogen rich surface, e.g. as produced under cathodic reduction in acidic solutions, is suitable for the covalent attachment of doxycycline and that covalently bound doxycycline is biologically effective.

The bioactivity of the biomolecule on the hydrided metal dental implant surface was assessed under *in vitro* and *in vivo* conditions and it was demonstrated that doxycycline coated dental implants surprisingly improved not only the biocompatibility of the implant measured on improved osseointegration, but clearly were able to promote healing of the soft tissue in proximity to the placed implant such as of gum tissue,.

Consequently, the present invention relates to a craniofacial and/or dental prosthetic device or implant, containing a metal material (A) selected from the group consisting of titanium or an alloy thereof, zirconium or an alloy thereof, tantalum or an alloy thereof, hafnium or an alloy thereof, niobium or an alloy thereof and a chromium-vanadium alloy, wherein surface parts of the metal material (A) are coated with a layer of a corresponding hydride material (B) selected from titanium hydride, zirconium hydride, tantalum hydride, hafnium hydride, niobium hydride and chromium and/or vanadium hydride, respectively, characterized in that the layer of hydride material (B) comprises one or more doxycycline(s) (C) associated therewith.

In one embodiment, a device or implant is related to, wherein the metal material (A) is titanium or an alloy thereof, preferably titanium.

Typically, the one or more doxycycline(s) (C) is present on the surface of the hydride material (B) or trapped in the hydride material of the device or implant, and in a presently preferred embodiment, the one or more doxycycline (C) is associated with surfaces that are in contact with soft tissue when the device is deployed in the body of a mammal. Nonetheless, also related to herein is a device or implant, wherein essentially all surface parts of the craniofacial and/or dental prosthetic device or implant are coated with a layer of a corresponding hydride material (B) and one or more doxycycline(s) (C) associated therewith.

In one embodiment, a craniofacial and/or dental prosthetic device or implant, such as a dental implant is described, wherein selectively the surface parts that are in contact with soft tissue, such as in gum tissue, when the craniofacial and/or dental prosthetic device or implant is deployed in the body of a mammal, are coated with a layer of a corresponding hydride material (B) and one or more doxycycline(s) (C) associated therewith.

The craniofacial and/or dental prosthetic device or implant, such as a dental implant as described in the present invention is particularly intended for use when the craniofacial and/or dental prosthetic device or implant is deployed in the body of patient with smoking habits, radiation therapy and/or poor oral hygiene.

In another, equally preferred embodiment, the craniofacial and/or dental prosthetic device or implant, such as a dental implant as described in the present invention, is particularly intended for use when the craniofacial and/or dental prosthetic device or implant is deployed in the body of patient with general poor bone density, poor quality bone, wound healing disruptions and/or high risk for infections.

Consequently, the present invention in one aspect relates to a craniofacial and/or dental prosthetic containing a metal material (A), wherein surface parts of the metal material (A) are optionally coated with a layer of a corresponding hydride material (B), characterized in that the layer of hydride material (B) or at least parts of metal material (A) comprises one or more doxycycline(s) (C) associated therewith, for use in promoting soft tissue healing and/or preventing inflammation and/or infection in soft tissue in proximity to the craniofacial and/or dental prosthetic device or implant, when deployed in the body of a high-risk patient, such as a patient with smoking habits, radiation therapy and/or poor oral hygiene, general poor bone density, poor quality bone, wound healing disruptions and/or high risk for infections.

In one embodiment, the doxycycline (C) is associated with the hydride material (B) in an amount from 1 picogram per mm2 to 1 mg per mm2, preferably in an amount from 0.1 nanogram to 100 microgram per mm2.

A device or implant as presently disclosed is typically a device or implant which replaces anatomy or restores a function of the body such as a craniofacial and/or dental prosthetic device or implant, such as a dental implant.

In the present context; craniofacial prostheses are prostheses made by individuals trained in anaplastology or maxillofacial prosthodontics who medically help rehabilitate those suffering from facial defects caused by disease (mostly progressed forms of skin cancer, and head and neck cancer), trauma (outer ear trauma, eye trauma) or birth defects (microtia, anophthalmia). They have the ability to replace almost any part of the face, but most commonly is the ear, nose or eye/eyelids. An ocular prosthesis and hair prosthesis can also be classified as craniofacial prostheses. Prostheses are held in place either by biocompatible drying adhesives, osseointegrated implants, magnets, or another mechanical form (although rare) such as glasses or straps. Prostheses are designed to be as similar as possible to the natural anatomy of each individual. Their purpose is to cover, protect, and disguise facial disfigurements or underdevelopments.

In the present context; a dental implant is a "root" device, usually made of titanium or an alloy thereof, used in dentistry to support restorations that resemble a tooth or group of teeth to replace missing teeth.

Virtually all dental implants placed today are root-form endosseous implants, i.e., they appear similar to an actual tooth root (and thus possess a "root-form") and are placed within the bone. The bone of the jaw accepts and osseointegrates with the titanium post. Osseointegration refers to the fusion of the implant surface with the surrounding bone. Dental implants will fuse with bone.

Dental implants can be used to support a number of dental prosthetic devices, including abutments, crowns, implant-supported bridges or dentures, such prostetic devices are typically in direct contact with soft tissue, such as gum tissue and/or oral mucosa. They can also be used as anchorage for orthodontic tooth movement. The use of dental implants permits undirectional tooth movement without reciprocal action.

A dental implant can comprise one or several parts. Single-piece dental implants can comprise and/or include a variety of prosthetic devise, including abutments, crowns, implant-supported bridges or dentures, such prostetic devices are typically in direct contact with soft tissue, such as gum tissue and/or oral mucosa.

One embodiment of the present invention consequently relates to dental implants either supporting, including, containing and/or comprising a dental prosthetic device, selected from the group consisting of abutments, crowns, implant-supported bridges or dentures, being in direct contact with soft tissue, such as gum tissue and/or oral mucosa, wherein essentially all surface parts of the craniofacial and/or dental prosthetic device or implant are coated with one or more doxycycline(s) (C).

Another embodiment of the present invention relates to dental implants either supporting, including, containing and/or comprising a dental prosthetic device, selected from the group consisting of abutments, crowns, implant-supported bridges or dentures, being in direct contact with soft tissue, such as gum tissue and/or oral mucosa, wherein, wherein selectively the surface parts that are in contact with soft tissue, such as gum tissue, when the craniofacial and/or dental prosthetic device or implant is deployed in the body of a mammal, are coated with one or more doxycycline(s) (C).

Also disclosed herein is a method for preparing a craniofacial and/or dental prosthetic device or implant as defined herein, said method comprising subjecting surface parts of the metal material (A) to an electrolysis treatment to form the layer of hydride material (B), said electrolysis treatment being carried out in the presence of one or more doxycycline(s) (C).

In addition, a craniofacial and/or dental prosthetic device or implant as defined herein can alternatively be prepared by other methods, such as dipping, spraying, nano-coating, plasma spraying, sputtering, deposition, chemical treatment with acids or alkali, hydrogen peroxide treatment, sol-gel, chemical vapor deposition, anodization, drying-on, and/or vaporizing of the one or more doxycycline(s) (C) to at least parts of the metal surface (A).

A use is further related to herein of a craniofacial and/or dental prosthetic device or implant as defined herein, for promoting soft tissue healing and/or preventing inflammation and/or infection in soft tissue in proximity to the craniofacial and/or dental prosthetic device or implant as defined herein, when deployed in the body of a mammal, in particular in a patient belonging to a high-risk patient group.

Further, Doxycycline is related to for use in promoting soft tissue healing and/or preventing inflammation and/or infection in soft tissue in proximity to a craniofacial and/or dental prosthetic device or implant as defined herein, when deployed in the body of a mammal, wherein said doxycycline is covalently bound selectively to the surface parts of the craniofacial and/or dental prosthetic device or implant as defined herein that are in contact with soft tissue, when the craniofacial and/or dental prosthetic device or implant as defined herein is deployed in the body of a mammal.

In the present context, the craniofacial and/or dental prosthetic device or implant as defined herein can be employed as a drug delivery device for a pharmaceutical composition comprising Doxycycline.

One aspect of the invention therefore relates to the use of Doxycycline for the preparation of a pharmaceutical composition for use in promoting soft tissue healing and/or preventing inflammation and/or infection in soft tissue in proximity to a craniofacial and/or dental prosthetic device or implant as defined herein, when deployed in the body of a mammal, wherein said doxycycline is covalently bound selectively to the surface parts of the craniofacial and/or dental prosthetic device or implant as defined herein that are in contact with soft tissue, when the craniofacial and/or dental prosthetic device or implant as defined herein is deployed in the body of a mammal.

In particular, a use of Doxycycline is herein related to, wherein the craniofacial and/or dental prosthetic device or implant as defined herein is deployed in a patient belonging to a high-risk patient group, such as patients with general poor bone density, wound healing disruptions or high risk for infections, such as a patient group that has poor success rates with commercially available implants, e.g. due to diabetes, smoking habits, radiation therapy and/or poor oral hygiene.

One aspect of the invention consequently relates to the use of Doxycycline for the preparation of a pharmaceutical composition for use in promoting soft tissue healing and/or preventing inflammation and/or infection in soft tissue in proximity to a craniofacial and/or dental prosthetic device or implant as defined herein, when deployed in the body of a mammal, wherein the craniofacial and/or dental prosthetic device or implant as defined herein is deployed in a patient belonging to a high-risk patient group, such as patients with general poor bone density, wound healing disruptions or high risk for infections, such as a patient group that has poor success rates with commercially available implants, e.g. due to diabetes, smoking habits, radiation therapy and/or poor oral hygiene.

Doxycycline is herein also related to for use in promoting soft tissue healing and/or preventing inflammation and/or infection in soft tissue in a patient carrying a craniofacial and/or dental prosthetic device or implant containing a metal material (A) selected from the group consisting of titanium or an alloy thereof, zirconium or an alloy thereof, tantalum or an alloy thereof, hafnium or an alloy thereof, niobium or an alloy thereof and a chromium-vanadium alloy, and wherein surface parts of the metal material (A) are coated with a layer of a corresponding hydride material (B) selected from titanium hydride, zirconium hydride, tantalum hydride, hafnium hydride, niobium hydride and chromium and/or vanadium hydride, respectively.

In one embodiment, Doxycycline is disclosed for use in promoting soft tissue healing and/or preventing inflammation and/or infection in soft tissue in a patient carrying a craniofacial and/or dental prosthetic device or implant containing a metal material (A) selected from the group consisting of titanium or an alloy thereof, zirconium or an alloy thereof, tantalum or an alloy thereof, hafnium or an alloy thereof, niobium or an alloy thereof and a chromium-vanadium alloy, and wherein surface parts of the metal material (A) are coated with a layer of a corresponding hydride material (B) selected from titanium hydride, zirconium hydride, tantalum hydride, hafnium hydride, niobium hydride and chromium and/or vanadium hydride, respectively, wherein said doxycycline is covalently bound to surface parts of the a craniofacial and/or dental prosthetic device or implant.

"Soft tissues", (i.e. non-mineralized tissues), can in the present context be used interchangeably with gingival tissue and/or gum tissue and may be defined as collagen or epithelium containing tissues, including skin and mucosa, such as oral mucosa, muscle, blood and lymph vessels, nerve tissues, glands, tendons, eyes and cartilage. In general the fraction and/or polypeptide fragments of the present invention can be used to promote healing or for manufacturing a pharmaceutical composition for promoting healing of a wound not only in skin and mucosa, but in any gingival tissue of the patient in need thereof.

The term "hard-tissue formation" in "mineralised tissue" may be summarised as the production by cells of an organic matrix capable of accepting mineral, with the activity of the enzyme alkaline phosphatase and a good blood supply prerequisites.

In the present context, the phrase a craniofacial and/or dental prosthetic device or implant includes within its scope a device intended to be implanted into the body of a vertebrate animal, in particular a mammal such as a human.

The device or implant according to the invention can be used for a number of purposes.

When the metal material (A) is an alloy of titanium, zirconium, tantalum, hafnium or niobium, it may be an alloy between one or more of these metal elements; or it may be an alloy containing one or more other metals such as aluminum, vanadium, chrome, cobalt, magnesium, iron, gold, silver, copper, mercury, tin or zinc; or both.

It is preferred that the metal material (A) is titanium or an alloy thereof, e. g. an alloy with zirconium, tantalum, hafnium, niobium, aluminum, vanadium, chrome, cobalt, magnesium, iron, gold, silver, copper, mercury, tin or zinc. In a particularly preferred embodiment, the metal material (A) is titanium.

The corresponding hydride material (B) is preferably titanium hydride.

The amount of Doxycycline (C) present on or in the hydride layer (B) of the parts of the prosthesis, device or implant coated with the hydride may vary within wide limits. Thus, the Doxycycline (C) associated with the hydride material (B) may be present in amounts ranging from as low from 1 picogram per mm² to as high as 1 mg per mm² of hydride-coated device or implant surface.

However, it is contemplated that most useful Doxycycline coatings will range from 0.1 nanogram to 100 microgram per mm2.

As indicated above, the method of the invention involves subjecting surface parts of the metal material (A) to a electrolysis treatment to form the hydride layer (B), said treatment being carried out in the presence of one or more Doxycycline(s) (C) as discussed above. It has been found that it is important that the conditions in the electrolyte (pH, ionic strength etc.) are such that the Doxycycline (C) has a net positive charge.

Consequently, the main concern for incorporation thereof in a hydride layer is stability under the conditions needed for bio-hydride preparation; I. e. an environment that supplies enough H+ ions for hydride preparation and at the same time keeps the net charge of the Doxycycline (C) positive. This mostly means that the electrolyte should have a high salt concentration and hence ionic strength; a comparatively high temperature, although preferably below any denaturing temperature of the Doxycycline (C); and a low pH.

Thus, the electrolyte may be any salt solution, preferably aqueous, e. g. a solution of sodium chloride, sodium sulphate, calcium phosphate, calcium chloride, phosphate buffered saline (PBS), saline, a salt solution mimicking physiological conditions, bicarbonates, carbonates etc., in which the Doxycycline (C) is dissolved. The ionic strength of the salt is typically 1M, but concentrations can be adjusted to as low as 0.01 M and as high as 10 M according to the chemical properties and concentration of the Doxycycline(s).

The temperature of the electrolyte containing the Doxycycline (C) may range from ambient (20°C) to as high as the boiling point of the electrolyte, typically around 100°C, although the use of temperatures in the upper part of this range clearly depends on the ability of the Doxycycline (C) to withstand such temperatures without damage. If the Doxycycline (C) can withstand it, an optimum temperature for the formation of hydride is around 80°C.

The pH of the electrolyte is typically adjusted to the desired pH by means of a strong acid, e. g. HCl, HF, H2504 etc. although it should be taken into account that a pH below 2 will produce an irregular, corroded implant surface on titanium while a pH above 2 conserves the original surface. The pH is adjusted according to the desired Hydride/ Doxycycline (C) ratio; Low pH produces an implant surface with a high hydride/ Doxycycline (C) ratio (=more metal hydride), whereas a high pH close to the pl of the Doxycycline (C) in question will produce a surface with a low hydride/ Doxycycline (C) ratio (=more biomolecules).

Accordingly, while any pH between 0 and 10 can be used, the preferred pH for hydride preparation is between 5 and 2, depending on the chemical characteristics and concentration of the Doxycycline(s), the electrolyte used and the preferred hydride/ Doxycycline (C) ratio. For higher hydride/ Doxycycline (s) ratios (=more hydride), adjust pH more acidic, for lower hydride/ Doxycycline (s) ratios (=more Doxycycline (s)) adjust pH closer to, but not above, PI Doxycycline (C). The only requirement is that there are hydrogen ions (H+) and positively charged Doxycycline (C) (Doxycycline (C)+, net charge) present in the electrolyte.

The concentration of the Doxycycline(s) (one or any combinations of two or more) in the electrolyte may vary over a very wide range depending on, if it is to be released or not from the hydride layer, stability in vivo, stability in the electrolyte, availability, optimal pH, etc., Thus, the concentration of the Doxycycline(s) in the electrolyte may be within the range of 1 pg to 50mg per milliliter. A preferred range is between 10 pg and 1 mg per milliliter, but the optimal Doxycycline(s) concentration should always be by finally determined in pilot experiments with each Doxycycline(s) or Doxycycline(s)-mix.

Also, the time span over which the electrolysis is performed may vary but chiefly influences the thickness of the hydride layer and hence the concentration of Doxycycline(s) in the hydride layer.

An electrolysis cell for use in the method of the invention may be of any conventional design but is typically a two-chamber cell without any conducting connections between the chambers except for the electrolyte. The metal implant to be hydride-modified is placed in the cathode (i.e. the negatively charged electrode) chamber whereas the anode (the positively charged electrode), typically made of carbon, is placed in a separate chamber. The electrolytes of each chamber are connected through a porous glass or porcelain filter allowing the current to pass unhindered but without any exchange of electrolytes between the two chambers. This is important because the products from the anode reaction, e. g. chloride or hypo-chlorites etc., could potentially interfere with the formation of the doxycycline -hydride layer or destroy or modify the doxycycline in the cathode electrolyte. The separation of the two cells also allows the use of a smaller cathode electrolyte volume and thus a more effective use of doxycycline(s) as well as the possibility to use a two-electrolyte system that allows optimization of the electrolytic process, e. g. one electrolyte optimal for doxycycline(s) on the cathode side and an electrolyte on the anode side which is optimized for the efficacy of the electrolysis per se (conductivity, avoiding toxic products, or even producing useful byproducts/coatings).

As indicated above, the temperature in the cathode cell (Teat) should be as high as possible with an optimum for hydride preparation at 80°C.

The electrolytic process itself also produces heat which can pose two problems; constituents of the electrolyte will evaporate so that the volume decreases and the ionic strength and the concentration of doxycycline(s) increase above the preferred range, and the increase in temperature might cause precipitation, coagulation, denaturation, degradation or destruction of the doxycycline (s) present. Therefore, the cathode compartment of the electrolysis cell is preferably equipped with a cooled lid for condensation of vaporized electrolyte and a temperature regulated radiator shell for stabilizing temperatures and volumes during electrolysis.

By adjusting current, charge and electrolyte composition it may also be possible to provide a favorable milieu for positive charge for most doxycycline(s). If not, a pulse field electrolysis set-up where the polarity of the electrodes is switching in controlled cycles during preparation of the biohydride layer could be one way to omit a negative net charge problem.

The power supply is typically a so-called current pump, i.e. a device delivering a constant current even if the resistance within the circuit varies. Although voltages between 0.1 and 1000 volts can be used, the voltage is typically below 10 volts. The current density during electrolysis is typically in the range of 0.1 mA to 1 A per square centimeter (cm²) of implant specimen. A preferred charge density is 1 mA/cm² although adjustments in the electrolyte, pH and temperature to increase doxycycline compatibility may command minor or major deviations from this value.

The duration of the process depends on several parameters such as the desired thickness of the bio-hydride layer, the composition and characteristics of the electrolyte, the characteristics of the doxycycline, the temperature and pH, the desired hydride/biomolecule ratio, the size of the implant specimen, the volume of the cathode electrolyte, the concentration of the doxycycline, etc. Thus, the duration of the process may be between 0.5 hours and several days. However, an optimal time-span is generally between 8 and 24 hours.

To monitor the bio-hydride process, a calomel electrode may typically be placed in the cathode chamber. When the hydride layer formation process at the cathode is optimal, a difference of-1 Volt is observed between the calomel electrode and the cathode. If the current differs much from this value, the process will be running under sub-optimal conditions and a change in the set-up should be considered. Furthermore, a temperature probe and a pH probe may typically be placed in the cathode chamber to monitor that the process is running within the desired pH and temperature limits. A stirring device such as a magnetic stirrer may also be applied in the cathode cell to continuously mix the electrolyte and keep the temperature homogenous and avoid variations in local ionic strength, pH and doxycycline concentrations.

After the electrolysis step, the now doxycycline/hydride-coated metal device or implant is immediately removed from the electrolyte and treated according to the requirement of the doxycycline (s) in question. Typically, the device or implant specimen is allowed to air-dry and is then packaged in a sterile, airtight plastic bag in which it is stored until use for implantation. However, some doxycycline(s) might be sensitive to drying, and consequently a wet storage system might be desired, e. g. like canning or storage in a fluid like saline or simply the electrolyte from the manufacturing process.
Although the electrolysis can be run under aseptic or even sterile conditions, the need for doing this may be avoided by including a sterilization step prior to use, using conventional methods such as ionizing radiation, heating, autoclaving, or ethylene oxide gas etc. The choice of method will depend on the specific characteristics and properties of the doxycycline (s) present in the metal hydride layer.

Prior to the electrolysis treatment, the device or implant should be thoroughly cleaned.

This may typically consist in the implant being mechanically pre-treated by electropolishing or sandblasting to modify surface structure if desired, and subsequently thoroughly cleaned using hot caustic soda followed by a de-greasing step, e. g. in concentrated trichloro-ethylene, ethanol or methanol, before being treated in a pickling solution, e. g. hydrofluoric acid, to remove oxides and impurities on the surface. After pickling the implant specimen is washed thoroughly in hot, double distilled, ion-exchanged water.

The invention is further illustrated by the following, non-limiting examples.

### LEGENDS TO FIGURES

**Figure 1****.** Surface analysis Doxy coated and untreated sample (SBAE) in comparison. The low resolution images show distinct differences in coloration of the coated sample in comparison to the untreated sample.
**Figure 2****.** FTIR of Doxy coated sample, one curve of pure Doxy powder (Doxycycline pure, purple line) and one spiked sample (blue line) are displayed in the figure.

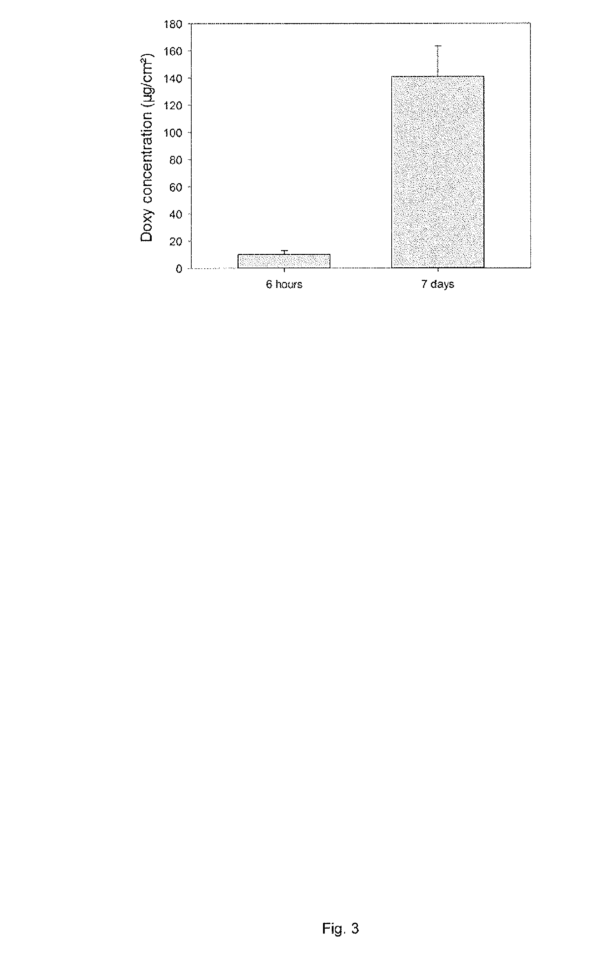

**Figure 3****.** UV-VIS release in a 40% ACN, 3% TFA solution over a total time of 7 days. Absorbance was measured at 263 nm.
**Figure 4****.** SIMS curves. Carbon depth profile over one Doxy coated and one TiZr SBAE coin (A). 1 H Depth profile over one Doxy coated and one control coin (B).
**Figure 5****.** XPS detail spectra of Doxy coated (green) and TiZr SBAE (red) samples for C 1 s, N 1 s and Ti 2p
**Figure 6****.** FE-SEM TiZr SBAE (A, C), Doxy coated (B, D) samples in two different magnifications. Pictures A and B show 10.000x magnification of the surface, pictures C and D show 30.000x magnification.
**Figure 7****.** In vitro after 14 days: ALP: Paired t-test; significant difference between Doxy electrocoated and untreated (P=0.038), data normally distributed and therefore displayed. (A)Toxicity, (B) Coll-I, (C) ALP, (D) OC: ANOVA on Ranks with Kruskal-Wallis, Multiple comparison with Dunn's Method; Data displayed as mean ± SEM. Significance levels; highly significant for P ≤ 0.01 displayed as **, significant differences for P ≤ 0.05 displayed as *.
**Figure 8****.** Analysis of wound fluid of implantation sites after retraction of TiZr SBAE, Hydrided and Doxy coated samples after time points of 4 and 8 weeks. Cytotoxicity was expressed by LDH activity (A) as a percentage of TiZr samples at 4 weeks, which were set to 100%. ALP activity was corrected for total protein (B). Values represent the mean ± SEM. Student t-test: (*) (p< 0.05) indicates significant differences compared to TiZr at each time point; (#) (p< 0.05) indicates significant differences compared to TiZr-H at each time point.

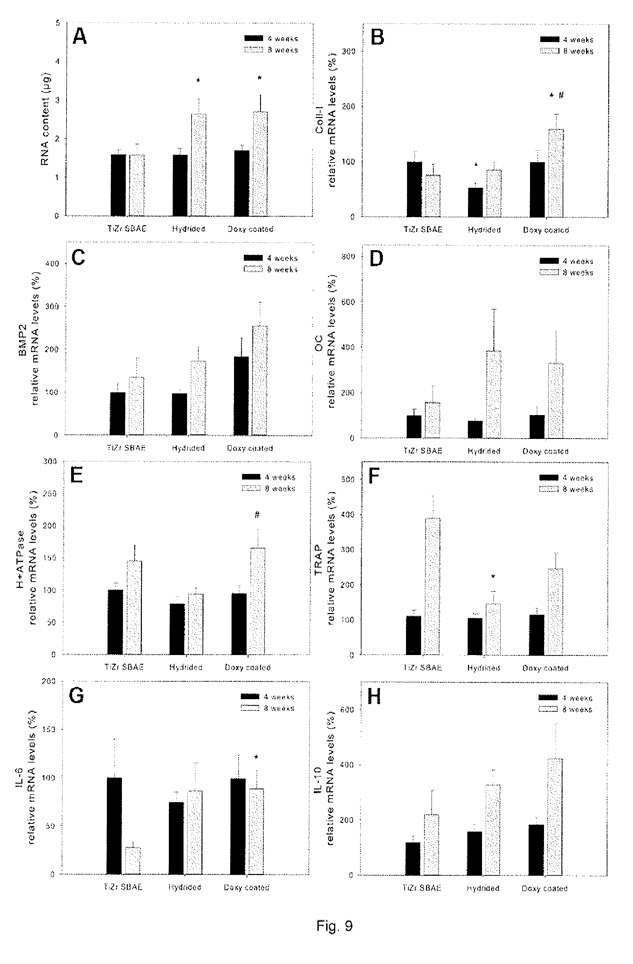

**Figure 9****.** 4 and 8 weeks total RNA content (A) gene expression of bone formation markers Coll-I, BMP2 and OC (B, C, D), bone resorption markers H+ATPase and TRAP (E, F), pro-inflammatory IL-6 (G) and anti-inflammatory IL-10 (H) markers at the bone implant interface. Data presents changes of target genes normalized with reference genes (GAPDH and 18S), expressed as percentage of TiZr SBAE samples at 4 weeks, which represent 100%. Values are displayed as mean ± SEM. Student t-test: (*) indicates p < 0.05 compared toTiZr SBAE at each time point, (#) indicates p < 0.05 compared to Hydrided at each time point.
**Figure 10****.** 2D BMD average of defects after retraction of TiZr SBAE, Hydrided and Doxy coated samples after healing times of 4 (A) and 8 weeks (B).

### EXPERIMENTAL SECTION

### Experiment 1:

### Material and Methods

### Sample preparation

This study employed coin shaped samples with a diameter of 6.25 mm and a height of 2 mm. The samples were made of titanium zirconium (TiZr) with a zirconium content of 13 % - 17 % zirconium, they were grit blasted and acid etched in hydrochloric and sulfuric acid (SBAE). After production, handling and packaging was done under nitrogen cover gas and the samples were stored in 0.9 % NaCl, making the surfaces comparable to the commercially available SLActive^{®} surface (Institute Straumann AG, Basel, Switzerland).

Before coating, the test coins were unpacked under laminar flow and washed for 5 minutes in an ultrasound bath with deionized reverse osmosis water.

The hydridation buffer for Doxycycline was a 2 M acetate buffer solution with a pH of 3 mixed at a volume of 200 ml of acetic acid (Fluka, Oslo, Norway) and sodium acetate (Sigma Aldrich, Oslo, Norway). This buffer was used directly for production of the hydrided coins from TiZr SBAE base material. For the doxycycline coated samples, 200 mg of Doxycycline were dissolved in the buffer to achieve a final concentration of 1 mg/ml. Doxycycline Hyclate was delivered by Yangzhou Pharmaceutical (Yangzhou Pharmaceutical Co Ltd, Jiangsu, China).

The coating was performed on a custom made setup, controlled by the software LabView, providing a constant direct current of 0.65 mA on individual channels for each sample. The current was controlled by a feedback loop with a PID controller. Thereby the sample coins were connected to the cathode of the system, whereas a platinum anode served as the counter electrode.

The TiZr coins were mounted on individual titanium holders, Teflon caps protected the interface between the holder and the coin from the electrolyte. After the process, the Doxycycline coated coins were dried under nitrogen gas flow and packed in containers filled with nitrogen prior to further analysis. Control samples were washed with ultrasound and dried under nitrogen in the same fashion as the Doxycycline coated samples. All samples were kept under cover gas before analysis as the exposure to air results in the attraction of environmental carbon, which subsequently lowers the signal intensity. Storage conditions were cold (< 10 °C), dry and dark.

### Optical Microscopy

A Leica DMRB Fluorescence Microscope (Wetzlar, Germany) was used for the optical assessment of the coin surface. Main focus was homogeneity and color shade differences in comparison to untreated TiZr SBAE coins. The microscope was used with external lightning and a 1.25 x objective, which in combination with the camera system added up to a total of 12.5 x magnifications. The software for image capturing was used with exposure time of 59 ms and ISO 100 sensitivity. Images were saved in bitmap format.

### Fourier transformed infrared spectroscopy (FTIR)

A PerkinElmer Spectrum 400 FT-IR/FT-NIR spectrometer (PerkinElmer, Waltham, MA, USA) was used to analyze the chemical composition of the surface. For analysis of the Doxycycline powder the ATR accessory was used, while the Doxycycline coated coins were measured with a diffuse reflectance (DR) accessory in order to compensate for the rough surfaces. The DR accessory is designed with an adjustable mirror to collect the scattered beams by the rough surface. After recording of a background spectrum from an untreated TiZr SBAE coin, the samples were scanned on three different spots each. The scans were executed on a wavelength between 4000 cm⁻¹ and 1000 cm⁻¹ in the mid infrared region. Every measurement was constituted of 8 individual measurements with a resolution of 8 cm⁻¹. After scanning all samples were background corrected, normalized and the Kubelka-Munk algorithm was applied to the datasets in order to quantify certain peaks with Beer's Law. Peak area quantification was carried out for two characteristic peaks of Doxycycline at 1454 cm⁻¹ and at 1613 cm⁻¹. Four reference coins were spiked with 10 µl of water dissolved Doxycycline to obtain a defined Doxycycline coverage on the reference sample. The amount of applied Doxycycline was 11.5 µg per coin. The solution covered the whole top coin surface and samples were dried under nitrogen flow. The spiked reference coins were measured in the same fashion as Doxycycline coated and control samples and their average signal intensity was calculated over all measurements for the characteristic peaks.

### UV- VIS Spectroscopy (UV-VIS)

A release study was executed to assess the total amount of Doxycycline released from the coins when submerged in liquid. The release was performed in a solution of 60 % acetonitrile and 3 % trifluoroacetic acid (ACN-TFA). Two time points with three Doxycycline coated samples per group were analysed after release in ACN-TFA. The release fluids were analysed after 6 hours and after 7 days in release with a Nano-Drop ND 1000 spectrometer (Thermo Fisher Scientific, Waltham, Massachusetts) in UV-VIS mode. A calibration curve was recorded at Doxycycline concentrations between 0.005 mg/ml and 0.25 mg/ml.

### Secondary Ion Mass Spectroscopy (SIMS)

Depth profiles of carbon and hydrogen were measured by secondary ion mass spectroscopy (Cameca IMS 7f, Paris, France) on the test coins. The analysis was carried out for ¹H and ¹²C ions. One control sample was included as background and a hydrided sample was used as control for carbon contamination. A 50 nA primary beam of 15 keV Cs⁺ was used for rastering over a surface area of 200 µm x 200 µm whereas negative secondary ions were collected from central part (67 µm x 67 µm) of the crater at room temperature. Doxycycline inclusion in the surface was detected by analysis of the depth profile of the ¹²C isotope.

The depth of each sputtering crater was analyzed by a blue light profilometer (SensofarPLµ 2300, Sensofar-Tech S.L., Terrassa, Spain), and the aspect heights calculated using an advanced software package (Sensomap, Sensofar-Tech S.L., Terrassa, Spain). The crater depth was measured with a 50 x objective (50 x PI, Nikon, Tokyo, Japan) over an extended topography of 2 x 2 images, each with a viewing area of 253 µm x 190 µm at 20 % overlapping. The result data from the SIMS measurement was calibrated according to the obtained crater depth.

### X-ray photoelectron Spectroscopy (XPS)

The XPS analysis was carried out on an Axis Ultra^{DLD} XP spectrometer (Kratos Analytical, Manchester, UK) using monochromatic Al Kα radiation (hv = 1486.6 eV). Survey spectra were recorded in a range between 1100 eV and 0 eV binding energy (BE). Detail spectra were acquired in the energy regions of O 1s, Ti 2p, C 1s, N 1s and Zr 3d. The instrument resolution was 1.1 eV for the survey scans and 0.55 eV for the detail scans. The analysis area was 300 µm x 700 µm.

The samples used for the XPS analysis were not stored under nitrogen cover gas after coating and washing. Samples were mounted on a sample bar with conductive carbon tape. The energy shift due to surface charging was below 1 eV based on the C 1 s peak position relative to established BEs, therefore the experiment was performed without charge compensation. All Bes were referenced to the C 1s peak 284.5 eV.

### Field Emission Scanning Electron Microscope (Fe-SEM)

The micro- and sub-micro structural analysis of the surface topography and morphology was performed with a FEI Quanta 200 FE-SEM (FEI Hillsboro, Oregon, USA). The samples were platinum sputtered prior to analysis and mounted on a 45° aluminum rack with conductive carbon tape. Images were acquired with a working distance between 5 mm and 7 mm. Acceleration voltages of 3 kV- 5 kV were applied to achieve magnifications of 10.000x and 30.000x. High vacuum (HV) operation mode was used for the examination of all samples.

### In vitro study

### Cell culture

The mouse osteoblastic cell line MC3T3-E1 was obtained from the German Collection of Microorganisms and Cell Cultures (DSMZ, Braunschweig, Germany). MC3T3-E1 cells were routinely cultured at 37 °C in a humidified atmosphere of 5% CO₂, and maintained in α-MEM supplemented with 10% fetal calf serum (FCS) and antibiotics (50 IU penicillin/ml and 50 µg streptomycin/ml). Cells were subcultured 1:50 before reaching confluency using PBS and trypsin/EDTA. All experiments were performed in the same passage of the MC3T3-E1 cells (passage 16).

The coins were placed in a well plate and 7x10³ cells were seeded on each well to study cell differentiation and cell toxicity. The same numbers of cells were cultured in parallel in plastic tissue culture dishes in all experiments. Trypan blue stain was used to determine total and viable cell number. For the experiments, MC3T3-E1 cells were maintained for 14 days on the implants in α-MEM supplemented with 10% FCS and antibiotics. Culture media was changed every other day. Culture media was collected after 24 hours to test the toxicity of the treatments and of the different implant surfaces (LDH activity). To study cell differentiation, cells were harvested at 14 days and collagen 1 (Coll-1), alkaline phosphatase (ALP) and osteocalcin (OC) gene expression were analyzed using real-time RT-PCR.

### Lactate dehydrogenase (LDH) activity in the culture media

The presence of LDH activity in the culture media was used as an index of cell death. LDH activity is determined spectrophotometrically after 30 min incubation at 25°C using 50 µl of culture media and adding 50 µl of the reaction mixture, by measuring the oxidation of NADH at 490 nm in the presence of pyruvate, according to the manufacturer's kit instructions (Cytotoxicity Detection kit, Roche Diagnostics). Positive control (100%) was cell culture media from cells seeded on plastic culture dishes and incubated with Triton X-100 at 1%. Negative control (0 %) was cell culture media from cells seeded on plastic culture dishes without any treatment.

### RNA isolation

Total RNA was isolated with Tripure (Roche Diagnostics), following the instructions of the manufacturer. RNA was quantified using a spectrophotometer set at 260 nm (Nanodrop).

### Real-time RT-PCR

Real-time PCR was performed for two *housekeeping genes*, 18S ribosomal RNA (18S rRNA), glyceraldehyde-3-phosphate dehydrogenase (GAPDH), and *3target genes,* ALP, collagen-I and osteocalcin. The primer sequences are detailed in Table 1.

**Table 1 Primers used in the real-time PCR of housekeeping and target genes.**

| **Gene** | **Primer sequence** |
|---|---|
| ALP | S 5'- AACCCAGACACAAGCATTCC -3' (SEQ ID NO:1) |
| | A 5'- GAGAGCGAAGGGTCAGTCAG -3' (SEQ ID NO:2) |
| Collagen-I | S 5'- AGAGCATGACCGATGGATTC -3' (SEQ ID NO:3) |
| | A 5'- CCTTCTTGAGGTTGCCAGTC -3' (SEQ ID NO:4) |
| Osteocalcin | S 5'- CCGGGAGCAGTGTGAGCTTA -3' (SEQ ID NO:5) |
| | A 5'- TAGATGCGTTTGTAGGCGGTC -3' (SEQ ID NO:6) |
| 18S rRNA | S 5'- GTAACCCGTTGAACCCCATT -3' |
| | (SEQ NO:7) |
| | A 5'- CCATCCAATCGGTAGTAGCG -3' (SEQ ID NO:8) |
| GAPDH | S 5'- ACCCAGAAGACTGTGGATGG -3' (SEQ ID NO:9) |
| | A 5'- CACATTGGGGGTAGGAACAC -3' (SEQ ID NO:10) |

The same amount of total RNA (0.25µg) from each sample was reverse transcribed to cDNA at 37 °C for 60 min in a final volume of 20µl, using High Capacity RNA to cDNA kit (Applied Biosystems). Each cDNA was diluted 1/4.
Real-time PCR was performed in the Lightcycler 480® (Roche Diagnostics, Mannheim, Germany) using SYBR green detection. Each reaction contained 7 µl Lightcycler-FastStart DNA Master^{PLUS} SYBR Green I (*containing Fast Start Taq polymerase, reaction buffer, dNTPs mix, SYBRGreen I dye and MgCl₂*), 0.5 µM of each, the sense and the antisense specific primers and 3µl of the cDNA dilution in a final volume of 10 µl. The amplification program consisted of a preincubation step for denaturation of the template cDNA (5 min 95 °C), followed by 45 cycles consisting of a denaturation step (10 s 95 °C), an annealing step (10 s 60 °C, except for ALP that was 5s 65°C) and an extension step (10 s 72 °C, except for ALP that was 12s 65°C). After each cycle, fluorescence was measured at 72 °C. A negative control without cDNA template was run in each assay.

Real-time efficiencies were calculated from the given slopes in the LightCycler 480 software using serial dilutions, showing all the investigated transcripts high real-time PCR efficiency rates, and high linearity when different concentrations are used. PCR products were subjected to a melting curve analysis on the LightCycler and subsequently 2% agarose/TAE gel electrophoresis to confirm amplification specificity, Tm and amplicon size, respectively.

Relative quantification after PCR was calculated by dividing the concentration of the target gene in each sample by the mean of the concentration of the two reference genes (housekeeping genes) in the same sample using the Advanced relative quantification method provided by the LightCycler 480 analysis software version 1.5 (Roche Diagnostics, Mannheim, Germany).

### In vivo rabbit study

### Animals

An animal study for assessment of the in vivo performance of SVS and Doxycycline was executed with 14 Grey Bastard Chinchillas. The animals were 6 months old and had a weight of 2.86 kg in average (Crl:CHB, Charles River Laboratories International, Inc., Research Models and Services, Sulzfeld, Germany). The animals were kept in cages during the experimental period the two days post-surgery, and one day prior to euthanization. Room temperature, humidity and diet were standardized. Sedation and anesthesia were done according to the Norwegian School of Veterinary Science, Laboratory Animal Unit; SOP on anesthesia of rabbits. The experiments had been approved by the Norwegian Animal Research Authority (NARA) and registered by this authority. The procedures have thus been conducted in accordance with the Animal Welfare Act of June 01^{st} 2010, No 94 and Regulation on Animal Experimentation of January 15^{th} 1996.

### Surgical procedures, sedation and euthanasia

Sedation of the rabbits as well as euthanasia and all surgical procedures were done as described by Rønold et al. (Rønold HJ, Ellingsen JE. The use of a coin shaped implant for direct in situ measurement of attachment strength for osseointegrating biomaterial surfaces. Biomaterials. 2002;23(10):2201-9. doi:10.1016/s0142-9612(01)00353-2.). The only difference to this process was the integration of a central defect, drilled into the bone marrow region, with a diameter of 3.5 mm as described by Haugen et al. (Haugen HJ, Monjo M, Rubert M, Verket A, Lyngstadaas SP, Ellingsen JE et al. Porous ceramic titanium dioxide scaffolds promote bone formation in rabbit peri-implant cortical defect model. Acta Biomater. (0). doi:10.1016/j.actbio.2012.09.009.).

### Wound fluid analysis

After implant detachment, wound fluid sampling and LDH analysis were carried out analogous to Haugen et al.. Furthermore an aliquot of 25 µl of wound fluid in duplicate was assayed for alkaline phosphatase (ALP) activity. ALP activity as well as total protein were assessed according to Monjo et al. (Monjo M, Rubert M, Wohlfahrt JC, Rønold HJ, Ellingsen JE, Lyngstadaas SP. In Vivo Performance Of Absorbable Collagen Sponges With Rosuvastatin In Critical-Size Cortical Bone Defects. Acta Biomater. 2009.).

### Gene expression

Total RNA was isolated from peri-implant bone tissue as described already elsewhere (Monjo M, Lamolle SF, Lyngstadaas SP, Rønold HJ, Ellingsen JE. In vivo expression of osteogenic markers and bone mineral density at the surface of fluoride-modified titanium implants. Biomaterials. 2008;29(28):3771-80.).

An amount of 280 ng of extracted RNA was reverse transcribed to cDNA at 42 °C for 60 min using High Capacity RNA-to-cDNA kit (Applied Biosystems, Foster City, CA), containing both oligo(dT) and random hexamers. Each cDNA was diluted 1/4 and aliquots were frozen (-20 °C) until the PCR reactions were carried out.

Real-time PCR was performed with a Lightcycler-480^{®} (Roche Diagnostics, Mannheim, Germany) using SYBR green detection. Real-time PCR was done for three housekeeping genes: 18S ribosomal RNA (18S rRNA), glyceraldehyde-3-phosphate dehydrogenase (GAPDH) and beta-actin, and nine target genes: bone morphogenetic protein 2 (BMP2), collagen type I (Coll-I), osteocalcin (OC), vacuolar type proton ATPase (H+-ATPase), tartrate-resistant acid phosphatase (TRAP), interleukin-6 (IL-6) and interleukin-10 (IL-10). Each reaction contained 7 µl Lightcycler-FastStart DNA MasterPLUS SYBR Green I (containing Fast Start Taq polymerase, reaction buffer, dNTPs mix, SYBRGreen I dye and MgCl2), 0.5 µM of each, the sense and the antisense specific primers and 3µl of the cDNA dilution in a final volume of 10 µl. The amplification program consisted of a preincubation step for denaturation of the template cDNA (10 min 95 °C), followed by 45 cycles consisting of a denaturation step (10 s 95 °C), an annealing step (8-10 s 60 °C) and an extension step (10 s 72 °C). After each cycle, fluorescence was measured at 72 °C (λex 470 nm, λem 530 nm). A negative control without cDNA template was run in each assay.

Real-time efficiencies were calculated from the given slopes in the Lightcycler-480^{®} software using serial dilutions, showing all the investigated transcripts high real-time PCR efficiency rates, and high linearity when different concentrations are used. PCR products were subjected to a melting curve analysis on the LightCycler and subsequently 2% agarose/TAE gel electrophoresis to confirm amplification specificity, Tm and amplicon size, respectively.

All samples were normalized by the geometric mean of the expression levels of β-actin and GADPH and fold changes were related to 4 weeks of implant placement using the mathematical model described by Pfaffl (Pfaffl MW, Horgan GW, Dempfle L. Relative expression software tool (REST©) for group-wise comparison and statistical analysis of relative expression results in real-time PCR. Nucleic Acids Res. 2002;30(9):e36-e.), as folows: ratio = E_{target}^{ΔCp target (mean control 4 weeks - sample)} / E_{reference}^{ΔCp target (mean control 4 weeks - sample)} , where Cp is the is the crossing point of the reaction amplification curve as determined by the Lightcycler-480^{®} software. Stability of reference genes was calculated using the BestKeeper tool (Pfaffl MW, Tichopad A, Prgomet C, Neuvians TP. Determination of stable housekeeping genes, differentially regulated target genes and sample integrity: BestKeeper-Excel-based tool using pair-wise correlations. Biotechnol Lett. 2004;26(6):509-15.). Thus, values were expressed as a percentage of TiZr (reference implant) at 4 weeks, which were set to 100 %.

### µ-CT analysis

After extraction of the coins and wound fluid sampling, the tibia samples were prepared for analysis and scanned with µ-CT as described elsewhere. Furthermore reconstruction and bone mineral density (BMD) calibration was done according to the same description (Monjo M, Lamolle SF, Lyngstadaas SP, Rønold HJ, Ellingsen JE. In vivo expression of osteogenic markers and bone mineral density at the surface of fluoride-modified titanium implants. Biomaterials. 2008;29(28):3771-80.).

VOI for each sample was chosen: a cylinder with a diameter of 3.5 mm and a height of 2.5 mm starting from the outer cortical bone defect (which was in contact with the implant surface) and towards the marrow space. This VOI is identical to the defect created in the cortical bone prior to placement of the samples. Furthermore analysis of the BMD devolution dependent on the distance to the implant surface (2D BMD) was executed with a diameter of 6.2 mm and a height of 2.5 mm. The threshold level was set from 65 to 255.

### Statistics

The statistical significance of the results from the in vitro study was assessed as follows. Analysis of the raw data was carried out with Excel 2007 (Microsoft, Redmond, USA) and SigmaPlot 11.0 (Systat Software Inc, Chicago, USA). All datasets were examined for parametric or non-parametric distributions (Shapiro-Wilk test). For the in vitro study, ANOVA on Ranks with Kruskal-Wallis multiple comparison was used for the assessment of OC. One way ANOVA was used for cytotoxicity Coll-I and osteocalcin. ALP was analyzed with paired t-test. The statistical analysis of the in vivo results was done with student t-test and Doxycycline coated samples were compared to TiZr SBAE and hydrided at each time point. Differences were considered significant (*) for p ≤ 0.05 and highly significant (**) for p ≤ 0.01, and labeled respectively. For differences compared to the hydrided group (#) for p ≤ 0.05 and highly significant (##) for p ≤ 0.01 were used, and labeled respectively All parametric data are presented as mean values ± standard deviation and non-parametric data as median ± interquartile range.

### Results

### Optical Microscope

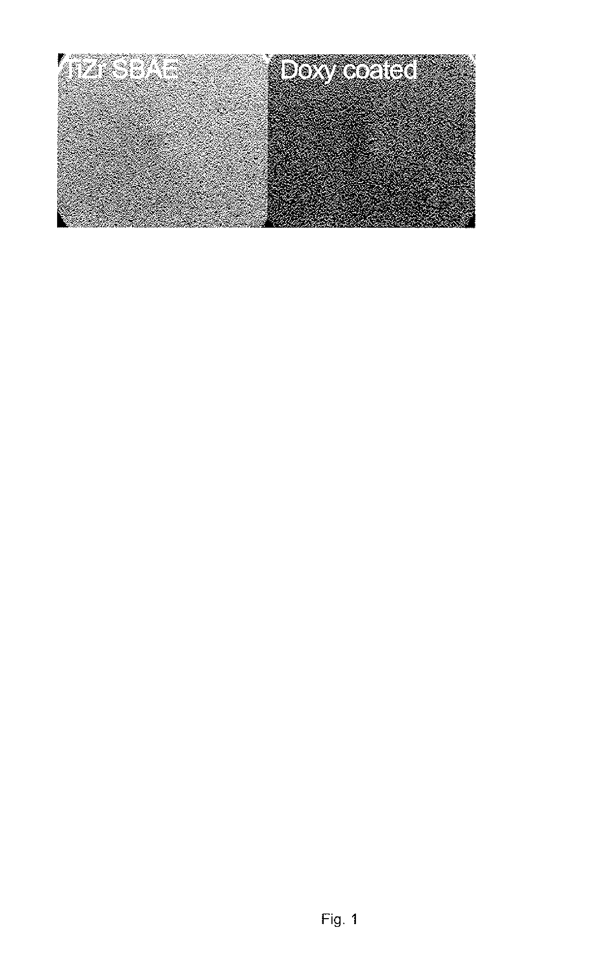

As the Doxycycline coated samples showed a different color after coating compared to the grey color of the control TiZr SBAE samples, a low resolution microscopy analysis was used to document the coin color. Figure 1 displays pictures of the surfaces recorded with a microscope. Doxycycline coated coins adopted a light brownish color after coating, which was equally distributed over the sample surface.

### FTIR

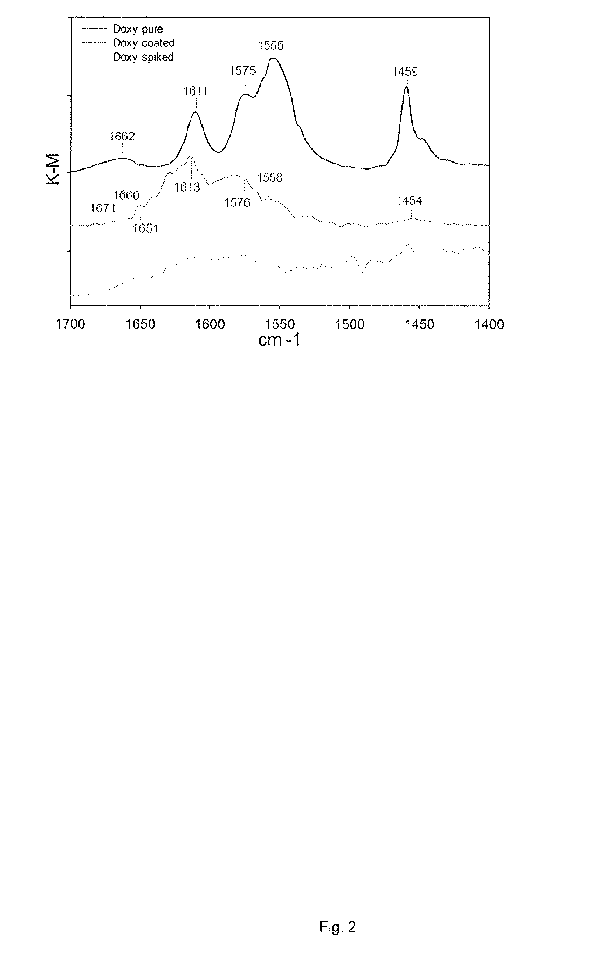

The FTIR was used to assess the integrity of Doxycycline on the surface after coating and to quantify its amount. All samples analyzed in FTIR showed stable expressions of the significant peaks at 1613 cm⁻¹ and 1454 cm⁻¹ as displayed in Figure 2. Furthermore peaks at 1558 cm⁻¹, 1576 cm⁻¹, 1651 cm⁻¹, 1660 cm⁻¹ and 1672 cm⁻¹ were detected. In comparison to the spiked sample, the coated samples showed higher absorbance values for both of the significant peaks (Figure 2). Spiked and coated samples showed a slight shift of the spectrum to higher wavelengths in comparison to the spectrum of pure Doxy. Pure Doxycycline showed mainly the absorption peaks at 1611 cm⁻¹, 1575 cm⁻¹, 1555 cm⁻¹ and 1459 cm⁻¹. In addition a weaker peak at 1662 cm⁻¹ appeared in this spectrum. The peaks at 1611 cm⁻¹, 1575 cm⁻¹ and 1555 cm⁻¹ were pronounced stronger in the spectrum of pure Doxycycline in comparison to the spiked and coated samples. Therein the peaks at 1575 cm⁻¹ and 1555 cm⁻¹ were expressed stronger than the ones at 1611 cm⁻¹ and 1662 cm⁻¹, relative to their respective spiked and coated counterparts. The uniformity of the four coated samples analyzed was visible in the spectra as well as the compliance of the coated coins with the Doxycycline powder.

The amount of Doxycycline on the coins was obtained by comparison of the samples absorbance values with the values of a spiked sample with a known concentration of Doxycycline (Table 2). The peak area underneath the significant peaks was calculated and set into relation to the known concentration on the spiked coin. The distribution was homogenous for the individual peaks, yet comparing the loading calculation for the peak at 1454 cm⁻¹ to the peak at 1613 cm⁻¹, a six times higher Doxycycline concentration was found for the peak at 1613 cm⁻¹ (Table 2).

**Table 2 Amount of Doxycycline over four coins - 3 measurements per coin. Spiked consists of 4 samples with 3 measurements each.**

| | **Doxy coated mean** | **spiked** |
|---|---|---|
| **Peak 1454 cm⁻¹ Doxy (µg/cm2)** | 6.9 | 11.5 |
| **Peak 1613 cm⁻¹ Doxy (µg/cm2)** | 45.1 | 11.5 |

### UV-VIS

As the quantification of loading with the FTIR provided a rather rough estimation, a release experiment was performed over a total period of 7 days. A first approach with water as release medium was not successful. Therefore, a solution containing 40% acetonitrile with 3% trifluoroacetic acid (ACN-TFA) was used as a release medium. With this method it was possible to detect a release of 10.27 µg/cm² after 6 hours and 140.97 µg/cm² after 7 days at a wavelength of 263 nm. The absorbance was measured at 263 nm as some peak shifting was detected in comparison to the literature.

### SIMS

SIMS measurements were obtained to confirm the integration of the doxycycline in the depth of the material. Figure 4 A shows the depth profile of a Doxycycline coated sample in comparison to a TiZr SBAE control sample and a hydrided sample. Doxycycline inclusion was detected up to a depth of 0.44 µm into the surface. At this sputtering depth, the concentration of the coated sample was approximately equal to the baseline carbon concentration of the bulk material. While the Doxycycline coated sample showed very high surface carbon levels, the control sample showed only slightly increased concentration in comparison to the baseline carbon level of the bulk material. The carbon concentration of the hydrided sample was elevated on the outer surface, but decreased rapidly.
Due to the proposed importance of hydrogen for the binding of Doxy, Figure 4 B displays the hydrogen profiles of a control and a Doxycycline coated sample. It could be seen that the control TiZr SBAE and the hydrided TiZr samples showed similar maximum concentration of hydrogen compared to the Doxycycline coated sample. Furthermore, the depth of the hydrogen layer was approximately 1 µm for all samples.

### XPS

The XPS analysis was performed to evaluate the chemical bonding of Doxycycline on the surface. Figure 5 shows the detail spectra of C 1s, N 1s and Ti 2p. It could be observed that the intensity of carbon and nitrogen peaks generally increased, whereas the peaks for titanium were strongly reduced on the Doxycycline coated sample in comparison to the control TiZr SBAE. The Ti 2p 3/2 peak was found at 459 eV and the Ti 2p 1/2 peak 5.5 eV higher. Whereas for control both Ti 2p peaks were well visible, the Ti 2p1/2 peak could not be observed on the Doxycycline coated sample. In addition, the carbon signal exhibited another peak at 286 eV and one at 287.5 eV. Nitrogen was present on both samples, exhibiting the major peak at 399.5 eV, and a smaller one at 401.6 eV. Furthermore the Doxycycline coated sample had another peak at 402 eV, which could not be observed on TiZr SBAE.

### SEM

A SEM study was conducted to optically assess the samples' surfaces. SEM images with 10.000x resolution revealed a smoother surface morphology for the Doxycycline coated samples compared to TiZr SBAE (Figure 7 A, B). Peaks on the Doxycycline coated surface appeared smoothened and sharp edges were not visible. Furthermore the surface showed no deep pits. In contrast, the control samples seemed rougher with sharp peaks on the edges. Despite those observations surface micro topography was similar for the two groups. The higher magnification (30.000x) images emphasized the differences between the samples (Figure 6 C, D). At this magnification, structures on Doxycycline coated sample expressed few topographical features and distinct structures were not visible. Edges were rounded and concave structures on the samples were smooth. In contrast, structures on the control sample were better visible, pits were identifiable and peaks showed sharp edges. In addition to that the Doxycycline coated samples showed white spherical structures on the sample surface that could not be observed on the control sample.

### In Vitro

The in vitro study was executed to evaluate the effect of the Doxycycline coated TiZr surfaces on the biological response of murine osteoblastic cells. Doxycycline coated and TiZr SBAE samples expressed generally low cytotoxicity values and differences between the samples were minimal and not significant. Cytotoxicity based on LDH activity, was generally lower in all TiZr surfaces compared to the tissue culture plastic reference group. ALP and OC were significantly increased on Doxycycline coated samples after 14 days, whereas Coll-I was slightly lower, without significance (Figure 7).

### In vivo

### Wound fluid analysis

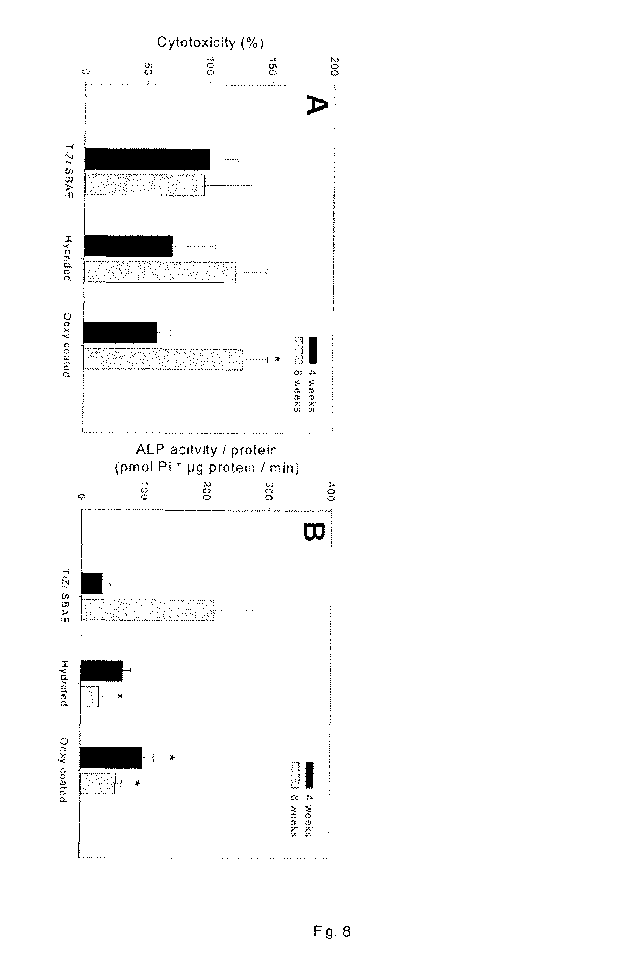

Wound fluid sampled from the implantation site after extraction of the implants was analyzed with regard to LDH activity as an indicator of cytotoxicity, ALP activity and protein content (Figure 8). Cytotoxicity according to LDH activity of TiZr at 4 weeks served as control and was set to 100 %. At the 4 week's time point the hydrided group expressed lower LDH activity than TiZr SBAE, while Doxycycline coated samples had lower activity compared to hydrided samples. Nevertheless, differences were not significant. At 8 weeks TiZr SBAE samples expressed slightly lower LDH activity compared to 4 weeks TiZr SBAE. In contrast, LDH activity for hydride and Doxycycline coated groups was higher than TiZr SBAE. This increase was found significant for the Doxycycline coated group (Figure 8 A).

The ALP activity was corrected for total protein content in the wound fluid to calculate specific ALP activity. The resulting factor was increased at 4 weeks for hydrided samples and significantly increase was found for Doxycycline coated samples compared to TiZr SBAE. As LDH activity is gradually decreasing during bone healing, hydrided and Doxycycline coated groups were found significantly lower than TiZr SBAE at 8 weeks (Figure 8 B).

### Peri implant bone tissue analysis

Total RNA in the 4 weeks samples was relatively similar for all groups. In contrast, at 8 weeks of healing time, the amount of RNA was significantly increased on hydrided and Doxycycline coated samples compared to TiZr SBAE (Figure 9 A).

The levels of Coll-I were decreased significantly for hydrided samples at 4 weeks in comparison to TiZr SBAE, while no significant difference was observed for the Doxycycline coated group. Coll-I an early marker of osteoblast differentiation, was significantly increased in Doxycycline coated samples, compared to TiZr SBAE and hydrided samples, after 8 weeks of healing time (Figure 9 B).

Levels of BMP-2 gene expression showed no significant differences at any of the time points, yet a trend of increasing BMP-2 expression with healing time was observed. Furthermore, at both time points, hydrided samples showed higher expressions than TiZr SBAE but lower expression than Doxycycline coated samples (Figure 9 C). Although OC gene expression did not change significantly, its expression was elevated after 8 weeks of healing time for hydrided and Doxycycline coated samples, compared to TiZr SBAE. Therein hydrided samples had higher OC gene expression levels than Doxycycline coated samples (Figure 9 D).

Both, H+-ATPase and TRAP, indicators of bone resorption, did not exhibit significant differences in mRNA levels for all examined groups at the 4 weeks. Nevertheless, H+-ATPase levels were significantly increased after 8 weeks in Doxycycline coated samples compared to the hydrided group and TRAP was found significantly lower than TiZr SBAE (Figure 9 E, F).

The inflammatory response to the tested surfaces was analyzed by gene expression of the pro-inflammation IL-6 cytokines and the anti-inflammatory IL-10 cytokines (Figure 9 G, H). IL-6 expression was increased significantly for Doxycycline coated samples after 8 weeks compared to TiZr SBAE, while no significant differences were found for IL-10. Nevertheless, IL-10 showed a trend of increasing gene expression after 8 weeks in Doxycycline coated samples compared to TiZr SBAE and hydrided.

### 2D BMD

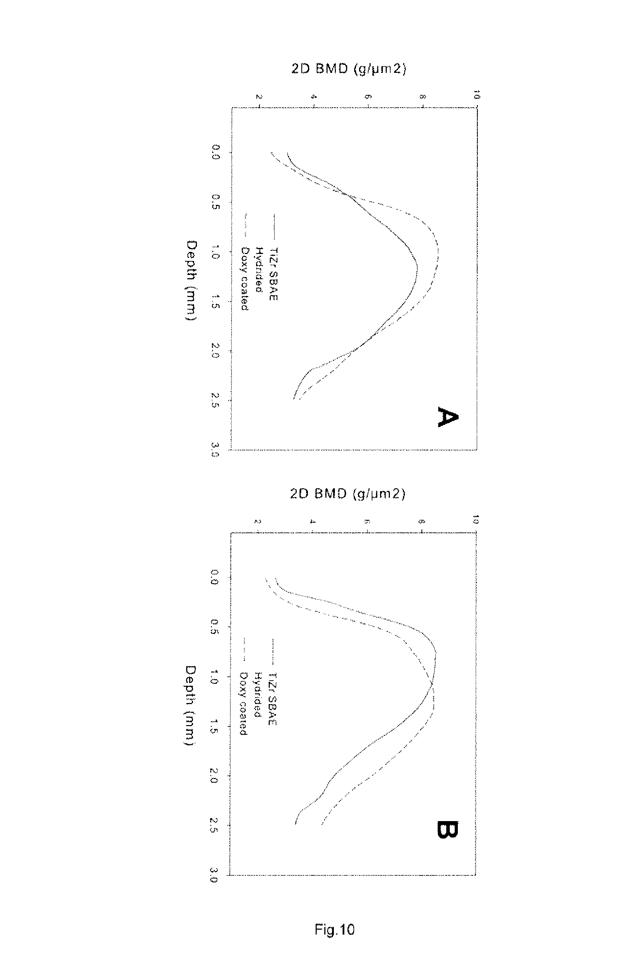

The devolution of BMD was analyzed with increasing distance from the sample surface in the defect regions of the tibia pieces (Figure 10). Highest depth integration and density of bone could be seen for the hydrided group after 4 weeks of healing time. The group with Doxycycline coated samples had similar maximum BMD values, but those were found at a lower distance from the implant compared to hydrided. Yet, both of the treated groups had higher BMD values compared to the TiZr SBAE control (Figure 10 A).
After 8 weeks, the hydrided group expressed lower maximum BMD values than hydrided and Doxycycline coated groups. Furthermore, the depth integration of bone was higher for Doxycycline coated samples than in TiZr SBAE and hydrided (Figure 10 B).

### 3D bone properties

The analysis of the bone in the 3.5 mm diameter volume of interest showed significant increase of bone surface in the Doxycycline coated group and the factor relating bone surface to tissue volume compared to the TiZr SBAE group. The latter parameter was also significant for the hydrided group. While the BMD showed only slight increase for hydrided and Doxycycline coated compared to TiZr SBAE, total bone volume increased for both treated groups approximately 16 % (Table 3).

**Table 3 3D parameter analysis of defect after 4 weeks. Statistical analysis with two way ANOVA. (*) indicates p < 0.05 compared toTiZr SBAE at each time point, (#) indicates p < 0.05 compared to same treatment at the other time point. Values are displayed as mean ± SEM.**

| | **BMD (g/cm³)** | | **Bone surface/ tissue volume (1/µm)** | | **Bone surface/ Volume ratio (1/µm)** | | **Bone surface (µm²)** | | **Bone volume fraction (%)** | | **Bone volume (µm³)** | |
|---|---|---|---|---|---|---|---|---|---|---|---|---|
| **weeks** | **4** | **8** | **4** | **8** | **4** | **8** | **4** | **8** | **4** | **8** | **4** | **8** |
| **TiZr SBAE** | 5.34 ±0.371 | 5.26 ±0.371 | 7.30 ±0.919 | 8.16 ±0.919 | 19.00 ±2.076 | 21.68 ±2.076 | 176.97 ±22.21 | 197.21 ±22.21 | 39.11 ±4.643 | 40.01 ±4.643 | 9.49 ±1.125 | 9.67 ±1.125 |
| **Hydrided** | 5.42 ±0.415 | 5.09 +0.415 | 11.47*, # ±1.027 | 6.59 # ±1.027 | 25.34 ±2.321 | 19.22 ±2.321 | 277.55 # ±24.832 | 159.26 # ±24.83 | 45.56 ±5.191 | 39.90 ±5.191 | 11.02 ±1.258 | 9.65 ±1.258 |
| **Doxy coated** | 5.46 ±0.415 | 5.72 ±0.415 | 11.69 * ±1.027 | 10.20 ±1.027 | 26.87 ±2.321 | 22.93 ±2.321 | 283.30 * ±24.832 | 247.57 ±24.832 | 45.34 ±5.191 | 49.63 ±5.191 | 10.99 ±1.258 | 12.04 ±1.258 |

After 8 weeks of healing time the increase in BMD amounted to 8.7 % and in bone volume to 24 % in the Doxycycline coated group, compared to TiZr SBAE . Bone surface, bone surface / volume ratio and bone surface / tissue volume were increased furthermore. In contrast to that, the hydrided group had lower values compared to TiZr SBAE for all parameters (Figure 10 B).

### Conclusion

The study demonstrated the efficiency of the process of coating a TiZr implant surface with Doxy. A hypothesis on the mechanism of binding based on a zwitterionic mechanism was postulated. It was possible to verify the integrity of the molecule on the surface and show its integration into the outer layer of the sample surface. In addition, the in vitro results demonstrated enhanced bioactivity of the Doxycycline coated samples after 14 days in comparison to TiZr SBAE, without negative effects on cell viability. Furthermore Doxycycline coated samples had positive effects on RNA content as well as Coll-I, BMP2 and OC mRNA levels in vivo after 8 weeks of healing time. Higher BMD values and total bone volume were detected in µ-CT after 8 weeks. Therefore it was concluded that Doxycycline coated surfaces positively influenced bone remodeling.

## Claims

1. A craniofacial and/or dental prosthetic device or implant,
a) containing a metal material (A) selected from the group consisting of titanium or an alloy thereof, zirconium or an alloy thereof, tantalum or an alloy thereof, hafnium or an alloy thereof, niobium or an alloy thereof and a chromium-vanadium alloy,
b) **characterized in that** surface parts of the metal material (A) comprise one or more doxycycline(s) (C).

2. A craniofacial and/or dental prosthetic device or implant,
a) containing a metal material (A) selected from the group consisting of titanium or an alloy thereof, zirconium or an alloy thereof, tantalum or an alloy thereof, hafnium or an alloy thereof, niobium or an alloy thereof and a chromium-vanadium alloy,
b)wherein surface parts of the metal material (A) are coated with a layer of a corresponding hydride material (B) selected from titanium hydride, zirconium hydride, tantalum hydride, hafnium hydride, niobium hydride and chromium and/or vanadium hydride, respectively,
c) **characterized in that** the layer of hydride material (B) comprises one or more doxycycline(s) (C) associated therewith.

3. A device or implant as claimed in claim 2, wherein the one or more doxycycline(s) (C) is(are) present on the surface of the hydride material (B) or trapped in the hydride material.

4. A device or implant as claimed in claim 2, wherein the one or more doxycycline(s) (C) is(are) covalently attached to the layer of hydride material (B).

5. A dental implant as claimed in any of the preceding claims, which supports a dental prosthetic device, selected from the group consisting of abutment, crown, implant-supported bridge or denture, being in direct contact with soft tissue, such as gum tissue and/or oral mucosa.

6. A dental implant as claimed in any of the preceding claims, which comprises a dental prosthetic device, selected from the group consisting of abutment, crown, implant-supported bridge or denture, being in direct contact with soft tissue, such as gum tissue and/or oral mucosa.

7. A dental prosthetic device as claimed in any of the preceding claims, which is selected from the group consisting of abutment, crown, implant-supported bridge or denture.

8. A device or implant as claimed in any of the preceding claims wherein the one or more doxycycline(s) (C) is(are) selectively associated with one or more surfaces that are in contact with soft tissue when the device or implant is deployed in the body of a mammal.

9. A device or implant as claimed in any of the preceding claims, wherein essentially all surface parts of the metal material of the craniofacial and/or dental prosthetic device or implant are at least partially coated with a layer of a corresponding hydride material (B) and one or more doxycycline(s) (C) associated therewith.

10. A device or implant as claimed in in any of the preceding claims, wherein the metal material (A) is titanium or an alloy thereof, preferably titanium.

11. A method for preparing a craniofacial and/or dental prosthetic device or implant as defined in any of claims 1-14, said method comprising subjecting surface parts of the metal material (A) to an electrolysis treatment to form the layer of hydride material (B), said electrolysis treatment being carried out in the presence of one or more doxycycline(s) (C).

12. A craniofacial and/or dental prosthetic device or implant as defined in any of claims 1-14 for use in promoting soft tissue healing and/or preventing inflammation and/or infection in soft tissue in proximity to the craniofacial and/or dental prosthetic device or implant, when deployed in the body of a mammal.

13. A craniofacial and/or dental prosthetic device or implant as defined in any of claims 1-14 for use in promoting soft tissue healing and/or preventing inflammation and/or infection in soft tissue in proximity to the medical prosthetic device or implant, when deployed in the body of a patient with smoking habits, radiation therapy, poor oral hygiene, general poor bone density, poor quality bone, wound healing disruptions and/or high risk for infections.

14. Use of a craniofacial and/or dental prosthetic device or implant as defined in any of claims 1-13 for promoting soft tissue healing and/or preventing inflammation and/or infection in soft tissue in proximity to the craniofacial and/or dental prosthetic device or implant, when deployed in the body of a mammal.

15. Doxycycline for use in promoting soft tissue healing and/or preventing inflammation and/or infection in soft tissue in proximity to a craniofacial and/or dental prosthetic device or implant as claimed in any of claims 1-13.

16. Doxycycline for use in promoting soft tissue healing and/or preventing inflammation and/or infection in soft tissue in proximity to a craniofacial and/or dental prosthetic device or implant, when deployed in the body of a mammalian patient, wherein said doxycycline is covalently bound selectively to the surface parts of the craniofacial and/or dental prosthetic device or implant that are in contact with soft tissue in said patient.

17. Doxycycline for use according to claim 16, wherein said doxycycline is covalently bound selectively to the surface parts of the craniofacial and/or dental prosthetic device or implant that are in contact with gum tissue and/or oral mucosa in said patient.

18. Doxycycline for use according to claims 16 or 17, wherein the craniofacial and/or dental prosthetic device or implant is deployed in the body of patient with smoking habits, radiation therapy, poor oral hygiene, general poor bone density, poor quality bone, wound healing disruptions and/or high risk for infections.


SEQUENCE LISTING

<110>  Straumann Holding AG

<120>  Dental Implants with Doxycycline for High Risk Patients

<130>  PS54593EP00

<160>  10

<170>  PatentIn version 3.5

<210>  1
<211>  20
<212>  DNA
<213>  Artificial sequence

<220>
<223>  Primer

<400>  1
aacccagaca caagcattcc                                                   20


<210>  2
<211>  20
<212>  DNA
<213>  Artificial sequence

<220>
<223>  Primer

<400>  2
gagagcgaag ggtcagtcag                                                   20


<210>  3
<211>  20
<212>  DNA
<213>  Artificial sequence

<220>
<223>  Primer

<400>  3
agagcatgac cgatggattc                                                   20


<210>  4
<211>  20
<212>  DNA
<213>  Artificial sequence

<220>
<223>  Primer

<400>  4
ccttcttgag gttgccagtc                                                   20


<210>  5
<211>  20
<212>  DNA

<213>  Artificial sequence

<220>
<223>  Primer

<400>  5
ccgggagcag tgtgagctta                                                   20


<210>  6
<211>  21
<212>  DNA
<213>  Artificial sequence

<220>
<223>  Primer

<400>  6
tagatgcgtt tgtaggcggt c                                                 21


<210>  7
<211>  20
<212>  DNA
<213>  Artificial sequence

<220>
<223>  Primer

<400>  7
gtaacccgtt gaaccccatt                                                   20


<210>  8
<211>  20
<212>  DNA
<213>  Artificial sequence

<220>
<223>  Primer

<400>  8
ccatccaatc ggtagtagcg                                                   20


<210>  9
<211>  20
<212>  DNA
<213>  Artificial sequence

<220>
<223>  Primer

<400>  9
acccagaaga ctgtggatgg                                                   20


<210>  10
<211>  20
<212>  DNA
<213>  Artificial sequence

<220>

<223>  Primer

<400>  10
cacattgggg gtaggaacac                                                    20